# EUROPÄISCHE PATENTSCHRIFT

(11) **EP 3 980 083 B1**
(45) Veröffentlichungstag und Bekanntmachung des Hinweises auf die Patenterteilung: **30.09.2026**
(21) Anmeldenummer: 20740509.3
(22) Anmeldetag: 10.06.2020
(51) Int. Cl.: A61L 2/183, A61L 2/206, A61L 2/10, A61L 2/04, A61L 2/06

(54) **VORRICHTUNG UND VERFAHREN ZUR REINIGUNG UND DESINFIZIERUNG VON GEGENSTÄNDEN SOWIE VERWENDUNG DER VORRICHTUNG UND DES VERFAHRENS UND DAMIT GEREINIGTE GEGENSTÄNDE**
METHOD AND DEVICE FOR CLEANING AND DISINFECTING OBJECTS
PROCÉDÉ ET DISPOSITIF DE NETTOYAGE ET DE DÉSINFECTION D'OBJETS

(30) Priorität: 10.06.2019 DE 202019103254 U; 26.03.2020 DE 202020101646 U; 09.06.2020 DE 202020103326 U
(43) Veröffentlichungstag der Anmeldung: 13.04.2022
(73) Patentinhaber: VESAN Management Holding GmbH, 57612 Eichelhardt (DE)
(72) Erfinder: CHANG, Sing-Hong Stefan, 52074 Aachen (DE); SÜMMERMANN, Gernot Jonathan, 52064 Aachen (DE)
(74) Vertreter: Neumann Müller Oberwalleney Patentanwälte PartG mbB
(86) Internationale Anmeldenummer: PCT/DE2020/100486
(87) Internationale Veröffentlichungsnummer: WO 2020/249161

(56) Entgegenhaltungen:
- EP-A1- 2 273 004
- WO-A2-2020/007398
- CN-A- 110 840 094
- US-A1- 2008 159 907
- US-A1- 2009 193 676
- US-B1- 6 845 569

## Beschreibung

Die Erfindung betrifft ein Vorrichtung und ein Verfahren zur Reinigung und Desinfizierung von Gegenständen, insbesondere für den Medizinischen und Laborbereich, nach dem Oberbegriff des 1. und10. Patentanspruchs sowie deren Verwendung und den damit gereinigten Gegenstand und betrifft vorzugsweise das Reinigen und Desinfizieren von Schutzkleidung und Schutzausrüstung wie Masken für den medizinisch-biochemischen Bereich. Darüber hinaus können mit dem nachfolgend beschriebenen Verfahren vorteilhaft aber auch Kleidungsstücke, Decken, Matratzen, und anderen textilen Ausstattungen in medizinischen Einrichtungen, aus dem persönlichen Nutzungs- oder Laborbereich desinfiziert werden.

Gemäß des Standes der Technik sind einige Desinfektionsverfahren mittels UV-C-Bestrahlung und Ozon-Generierung bekannt, die jedoch viele Nachteile aufweisen.

Aus der Druckschrift DE 698 01 450 T2 ist ein Verfahren zur Desinfektion von Flüssigkeiten und Gasen sowie Vorrichtungen, zur Verwendung des genannten Verfahrens bekannt. Die Druckschrift betrifft insbesondere Verfahren zur Desinfektion von Flüssigkeiten und Gasen durch Licht, welches durch Lichtleiter in die Flüssigkeiten und Gase gestrahlt wird. Das Licht wird in Form von Ultraviolettstrahlung (UVA, UVB, UVC) verwendet, da sich diese Strahlung zum Abtöten von Bakterien oder mikroskopischen schädlichen Mikroorganismen besonders eignet. Das Licht kann zudem aus dem sichtbaren Bereich des Spektrums stammen. Das Verfahren umfasst das Verteilen mindestens eines Lichtleiters in einem Bereich, der die zu desinfizierenden Flüssigkeiten oder Gase enthält. Es folgt ein Ausrichten mindestens einer Strahlungseinheit mit einer Lichtquelle hoher Intensität und anschließendes Bestrahlen der Flüssigkeit oder des Gases über einen vorbestimmten Zeitraum. Die Strahlungseinheit ist in Form eines Lasers ausgebildet.

In der Druckschrift EP 2 273 004 B1 wird ein Schrank zum Reinigen von Kleidung beschrieben, wobei der Schrank einen Reinigungsraum für die zu reinigende Kleidung und Mittel zur Generierung von Ozon aus Luft aufweist. Das Ozon wird in den Reinigungsraum eingebracht und mit der Kleidung in Kontakt gebracht. Der Schrank weist einen Geräteraum auf, in dem Ozon für die Reinigung der Kleidung generiert und der Kleidung zugeführt wird. Der Geräteraum ist durch eine Wand von dem Reinigungsraum getrennt ausgebildet.

Eine Vorrichtung zum Sterilisieren von Schuhen ist aus der Druckschrift US 4,981,651 A bekannt. Die Vorrichtung weist eine längliche Lampe zur Abgabe von ultravioletter Strahlung in einem Gehäuse sowie ein Heizelement und einen Ventilator auf. Die Vorrichtung ist in einen Schuh einbringbar, wobei mittels der Lampe und des Heizelements eine Sterilisation der Schuhsohle erreicht wird. Das Wachstum von Pilzen und Bakterien wird gestoppt. Es wird jedoch nur eine Luftzirkulation innerhalb der UV-Strahlungsquelle, jedoch nicht in der darum befindlichen Kammer beschrieben. Eine Absaugung und ein Filter werden nicht offenbart.

Eine ähnliche Vorrichtung wird in US 2016/0339126 A1 (D1) beschrieben. Darin sind in einem Gehäuse UV-Quellen angeordnet, welche in zu reinigende Textilien zumindest teilweise eingebracht werden. Eine Absaugung ist hier nicht vorgesehen.

Aus US 3 877 152 A geht eine Kammer zum Behandeln von Textilien mit einer Luftzufuhr, einer Heizquelle und einer UV-Strahlungsquelle hervor, mit welcher Ozon erzeugt wird. An die Kammer kann ein Vakuum angelegt werden.

Auch das Dokument CH 359 113 A beschreibt eine Kammer, an die ein Vakuum angelegt und dadurch ein Luftstrom erzeugt wird. Das Vakuum dient zum Abführen des Ozons und der von der Wäsche gelösten Schmutzpartikel.

Aus der Druckschrift DE 10 2012 209 823 A1 sind ein Verfahren und eine Vorrichtung zur Reinigung von Textilien bekannt wobei diese durch eine Öffnung in die Kammer eines Haushaltsgerätes eingelegt werden. In die Kammer des Trockners wird Luft eingebracht. Die Luft wird mit UV-C Licht, 100 bis 280nm, bestrahlt, wobei ein dafür vorgesehener Ozongenerator außerhalb der Kammer angeordnet ist. Es ist mit dieser Lösung keine effiziente Reinigung der Innenbereiche der Kleidungsstücke oder der Oberfläche anderer Materialen bzw. Gegenstände möglich.

Dokument DE 10 2007 037 984 A zeigt eine Vorrichtung mit einer Luftzirkulation und einer Ozonquelle, die in die Textilien eingebracht werden kann. Dabei kann die Luft das Gewebe durchströmen. Durch eine Abscheidevorrichtung werden Ozon und Schmutzpartikel entfernt. Die UV-Quelle befindet sich am kammerseitigen Ende der Luftzufuhr, wobei jedoch der Luftstrom nicht für mehrere UV-Strahlungsquellen aufgeteilt wird. Weiterhin ist keine Druckänderung vorgesehen.

Aus der Druckschrift US 2009/0193676 A1 ist eine Vorrichtung zur Trocknung von Schuhen bekannt, welches jedoch die Ozonerzeugung und die UV-Bestrahlung voneinander trennt, da ein Ozongenerator in einer separaten Kammer außerhalb des Gehäuses, in dem die zu reinigenden Gegenstände angeordnet sind, befindet..

Aus der US 2008/159907 A1 ist eine Vorrichtung bekannt, die einen Schrank umfasst, der eine Kammer definiert und wobei eine Schublade verschiebbar in der Kammer positioniert ist und zwischen einer offenen Position und einer geschlossenen Position beweglich ist. Die Schublade definiert ein Fach, das für die Aufnahme eines Gegenstandes, z.B. von Kleidung (Sweatshirt, Schuhe, Jacke) konfiguriert ist. In der geschlossenen Position ist die Schublade so konfiguriert, dass der Luftstrom aus dem Fach eingeschränkt wird. Die Vorrichtung umfasst einen Ozongenerator um Ozon zu erzeugen. Dieser ist jedoch nicht in der Schublage bei den zu desinfizierenden Gegenständen positioniert, sondern in einem separaten Maschinenraum.

Es ist mit den vorgenannten Lösungen keine effiziente Reinigung und Desinfizierung der Kleidungsstücke oder der Oberfläche anderer Materialen bzw. Gegenstände wie Decken, möglich, da die Durchdringung mit dem Ozon nicht vollständig gewährleistet werden kann.

Die Druckschrift CN110840094 offenbart einen Kleiderschrank mit Kleiderstange, mit einer UV Lampe und einem Gebläse zur Luftanströmung und Ozonerzeugung. Der Luftstrom wird jedoch nicht in das innere der Kleidung und somit nicht in das Innere der zu reinigenden Gegenstände geleitet. Es wird weiterhin nichts zu Druck- und Temperaturparametern in der Vorrichtung während des Reinigungsprozesses ausgesagt.

Aus der Druckschrift US6,845,569 B1 ist eine Vorrichtung zur Trocknung von Schuhen bekannt, welche eine Vielzahl von Leitungen mit einer gemeinsamen Luftzufuhr aufweist. Die Luft wird aus einer separaten Kammer zugeführt, in welcher eine Desinfektions-Desodorierungs-Anordnung positioniert ist, die ein Luftgebläse 41, eine Ozonlampe 42, einen Anionenluftgenerator 43 beinhaltet. Die einzelnen Rohre sind jeweils mit einem offenen Ende versehen, an dem eine Aufnahme für einen Schuh angeordnet ist, wobei durch die Rohröffnung (Durchströmöffnung) Luft in den Schuh strömt. Durch die Anordnung der Ozonlampe in der separaten Kammer wird keine optimale Desinfektion der Schuhe gewährleistet. Das Trocknen der Schuhe erfolgt bei 20 bis 40°C für bis zu 60 Minuten. Zu dem Druck in der Vorrichtung werden keine Angaben gemacht.

Auch in der Druckschrift DE 2020/007398 A1 wird eine gattungsgemäße Vorrichtung beschrieben, bei welcher jedoch an einem Grundkörper immer nur zwei Durchströmöffnungen vorgesehen sind und der Grundkörper fest in der Vorrichtung sitzt oder nur gemeinsam mit einem Ausschub aus der Vorrichtung fahrbar ist, so dass das Bestücken des Grundkörpers mit den zu reinigenden Gegenständen schwierig ist.

In der Druckschrift DE 11 2007 000 615 T5 wird ebenfalls eine Vorrichtung zum Sterilisieren von menschlichem Schuhwerk beschrieben, welche eine Lichtquelle aufweist, die Strahlung in einem Wellenlängenbereich emittiert, die das Schuhwerk sterilisiert, indem das Wachstum von Mikroorganismen, die in einem inneren Bereich des Schuhwerks vorhanden sind, gehemmt wird oder diese zerstört werden; einen Träger für die Lichtquelle, um sie in eine Position zu setzen, um die Strahlung auf den inneren Bereich des Schuhwerks zu richten; und eine Lichtblockierung, die während der Sterilisation verhindert, dass die Strahlung eine Person schädigt, die nahe dem Schuhwerk angeordnet ist. Weiterhin betrifft diese Lösung eine Vorrichtung zum Sterilisieren von menschlichem Schuhwerk mit einer Öffnung, in die der Fuß einer Person eingesetzt wird, um auf dem Schuhwerk zu stehen, umfassend: eine Lichtquelle, die Strahlung in einem sichtbaren Wellenlängenbereich emittiert, die das Schuhwerksterilisiert, indem das Wachstum von Mikroorganismen, die in einem inneren Bereich des Schuhwerks vorhanden sind, gehemmt wird; und einen Träger für die Lichtquelle, um sie in eine Position zu setzen, um die Strahlung in den inneren Bereich des Schuhwerks zu richten, um es zu sterilisieren.

Nachteil dieser beiden vorgenannten Lösungen ist, dass anhaftende Verunreinigungen nicht entfernt werden.

In einem Interview von Sabine Elsässer mit Stefan Chang, Gründer von Hygenator und Erfinder der RefresherBoxx, am24. Mai 2018 (2018-05-24), Ref. s. URL:https://www.startupvalley.news/de/stefanchang-hygenator-refresherboxx/ bzw. gemäß

XP055667315, wird ausgesagt, dass diese Box mit einer intelligenten und einzigartigen Kombination aktuellster Technologien (UV_C Licht, Ozon, Temperaturvariation, Unter- und Überdruck) arbeitet. Durch die rein physikalische Reinigungsmethode werden Gestank produzierende Bakterien, wie z.B. Propionibacterium oder Staphylococcus Epidermis denaturiert. Das heißt ihre Zellwände werden zerstört und die Bakterien somit abgetötet. Mit Hilfe von Druck in der Box werden die denaturierten Bakterien, Pilze und Feuchtigkeit herausgetragen. Darüber hinaus wirkt die RefresherBoxx antibakteriell und antimykotisch, sodass Pilzinfektionen bekämpft werden und diesen vorgebeugt wird.

Weiterhin wurde zum Thema in den Aachener Nachrichten am 8. Mai 2018 (2018-05-08) der Artikel veröffentlicht: "Aachen: Junger Erfinder aus Aachen sorgt für frische Schuhe", s. auch gemäß XP055663871 und unter
URL:https://www.aachener-nachrichten.de/lokales/aachen/junger-erfinder-aus-aachen-sorgt-fuerfrische-schuhe aid-24508537. Daraus ist jedoch lediglich für die Desinfektion von Schuhen entnehmbar, Zitat "mit einer UV-Lampe kann man Ozon generieren, die Feuchtigkeit wird entfernt, zugleich aber auch Pilze und Mikroorganismen" - ferner - ..... "Ein Filter saugt alles auf. Der Schuh wird nicht beschädigt."

Wie diese in den Artikeln angedeuteten Technologien im Einzelnen zusammenwirken und wie die Box konstruktiv ausgestaltet ist, kann den beiden vorgenannten Veröffentlichung aber nicht entnommen werden.

Aufgabe der Erfindung ist es, eine Vorrichtung und ein Verfahren zur schonenden Reinigung und insbesondere praktisch 100%ige Desinfizierung wenigstens eines Gegenstandes, insbesondere von Textilien wie Kleidung, Schutzkleidung, Mundschutz, Kittel, Mäntel, Handschuhen u.s. für den Medizinischen und Laborbereich bereitzustellen, wobei insbesondere eine umweltschonende Beseitigung von Bakterien und Viren ohne die Anwendung von chemischen Zusätzen ermöglicht wird und die Vorrichtung einen einfachen konstruktiven Aufbau aufweist.

Insbesondere war Aufgabenstellung eine Vorrichtung und ein Verfahren bereit zu stellen, um medizinische Schutzausrüstung rasch wieder zu verwenden. Dieser Wiederverwendung kommt in Zeiten von Epidemien und Pandemien eine besondere Bedeutung zu, insbesondere wenn aus logistischen Gründen z.B. in schlecht zugänglichen Gebieten und bei Liefer- und Produktionsengpässen ein akuter Mangel an Schutzausrüstung besteht.

Diese Aufgaben werden durch ein Verfahren und eine Vorrichtung mit den Merkmalen der Ansprüche 1 und 10 gelöst.Vorteilhafte Ausgestaltungen ergeben sich aus den Unteransprüchen.

Die Vorrichtung zur Reinigung und Desinfizierung wenigstens eines Gegenstandes wie Kleidung und/oder Schutzmasken, insbesondere für den medizinischen oder Laborbereich, weist eine von einem Gehäuse umgebene Kammer aufweist, in der ein mindestens ein Grundkörper zur Aufnahme des Gegenstandes angeordnet ist, wobei der Grundkörper einen Hohlraum und an seinem Außenumfang eine oder mehrere Durchströmöffnungen zum Hohlraum aufweist, wobei im Bereich mindestens einer Durchströmöffnung mindestens eine UV-Licht abstrahlende und Ozon erzeugende UV-Strahlungsquelle angeordnet ist und dass in den Hohlraum des Grundkörpers mindestens eine Luftzuführung führt, wobei die Luft aus dem Hohlraum des Grundkörpers durch den Durchbruch an der UV-Strahlungsquelle vorbei in den Gegenstand leitbar ist, wobei eine Vielzahl von sich gegenüberliegenden und übereinander angeordneten Aufnahmen in Form von Vorsprüngen vorgesehen sind oder wobei der Grundkörper in Form eines rohrförmigen Hohlkörpers ausgebildet ist, an dessen Umfang sich in einer Ebene jeweils mehrere Vorsprünge erstrecken und mehrere Vorsprünge übereinander angeordnet sind und der an seinem unteren Ende drehbar gelagert ist. Vorteilhafter Weise sind mit der erfindungsgemäßen Lösung Bakterien und Viren gemäß der nachfolgenden Tabelle 1 aus Gegenständen entfernbar.

**Tabelle 1 - Blindtabelle zu behandelbaren, insbesondere pathogenen Mikroorganismen (hier Beispiele teilw. mit engl. Bezeichnungen)**

| | Name | Name |
|---|---|---|
| | Anthraces | Tuberculosis |
| | Diphtheria | Vibrio Choleria |
| | Clostridium Botulism | Pseudo monas Bacteria |
| Bacteria | Tetanus | Salmonella |
| | Dysentery Bacillus | Fever Bacteria |
| | Cobibacillu | Bacillu Typhi murium |
| | Hook-side Pylon Bacillus | Shigella |
| | Legionella | Staphylococcus |
| | Micro co | Streptococcus |
| Virus | Adenovirus | MERS-CoV |
| | Phagocyte Cell Virus | Polio Virus |
| | Coxsackie Virus | Rota virus |
| | ECHO Virus | SARS-Familie, z.B. Covid-19 |
| | ECHO Virus 1 | Tobascco Mosaic Virus |
| | Influenza Virus | Hepatitis B Virus |
| Mold Spore | Aspergillus Niger | Soft Spores |
| | Aspergillus | Penicillium |
| | Dung Fungi | Penicillium Chrysogenum |
| | Mucor | Other Fungi Penicillium |
| Fish Desease | Fung 1 Disase | Infectious Pancreatic Necrosis |
| | Leukodermia | Hemorrhagic |

Als Gegenstand bzw. Gegenstände werden die in der nachfolgenden Tabelle 2 aufgezählten Produkte bzw. Einheiten zusammengefasst / bezeichnet:

**Tabelle 2 - mit der Erfindung zu reinigende bzw. zu desinfizierende Gegenstände insbesondere Materialien für den medizinischen, veterinärmedizinischen oder Laborbereich**

| | | |
|---|---|---|
| Alltagsgegenstände | Aus textilen Materialien, wie | Polymere-basierte Gegenstände, z.B. aus Kunstfasern, |
| | Kunstfasern, Kaschmir, Naturfaser, Seide, Samt, Wolle | PE-basierte Kleidung und Gegenstände, polymerbasierte Schutzausrüstung, Mundschutz |
| | aus Leder | |
| | Schuhwerk | Kleidung wie Kittel, Strümpfe Handschuhe, Jacken, Mantel, Hosen, |
| | Sportausrüstung | Trikots, Schuhe, Sportschuhe Sportbekleidung |
| Spezielle Ausrüstungen | Feuerwehrausrüstung | |
| | Laborkittel / Laborschutzausrüstung / Laborausstattung zur Biochemie, Laborgeräte, z.B. im Bereich Chemie, Biochemie bzw. Mikrobiologie | |
| | Rettungskraftausrüstung | |
| | Schutzausrüstungen | |
| Med. Gegenstände und Geräte | Medizinische Schutzausrüstungen | |
| | Atemschutzmasken; | |
| | OP-Bedarf, Ausrüstung in der Intensivpflege, Pflegedienst Ausrüstung, insbesondere im ambulanten Bereich | |

Im medizinischen Bereich betrifft die Reinigung insbesondere Schutzkleidung z.B. Mundschutz, Handschuhe, Kittel, Überstülpkappen für Kopf und Schuhe aus Textilfasern oder Polymeren gemäß Tabelle 2.

Derartige Gegenstände bzw. Textilien und Kleidungsstücke aus dem medizinischen oder biochemischen Krankenhaus-, Entwicklungs- oder Forschungsbereich gemäß Tabelle 1, insbesondere aus Sicherheitsbereichen, werden bisher vielfach nur einmal verwendet oder müssen aufwendig mit i.d. Regel aggressiven Desinfektionsmittel in einer Waschmaschine und anschließendem Trocknen in einem Wäschetrockner gereinigt, um Verschmutzungen, Keime, Viren, Bakterien und Pilze bzw. deren Sporen gemäß Tab. 2 zu entfernen.

Ferner werden im Krankenhaus allgemein solche Schutzausrüstungen mit hohen Temperaturen und unter Einsatz von stark reaktiven Chemikalien wie z.B. Biociden und vielfach zu Lasten der Materialeigenschaften desinfiziert.

Verfahrensgemäß erfolgt die Reinigung und Desinfizierung und die Eliminierung von pathogenen Organismen gemäß Tabelle 1 in oder auf Gegenständen gemäß Tabelle 2, welche z.B. aus textilem Material besteht, insbesondere eines Kleidungsstücks wie, Kittel, Jacken, Mäntel, Mundschutz, Handschuhen und dergleichen für den medizinischen oder veterinärmedizinischen Bereich oder den Laborbereich, Forschungslabore wie z.B. Virologie, Impfstoff-Entwicklungslabore dadurch, dass der der Gegenstand in der Kammer mittels eines Aufnahmeelements aufgenommen wird und in der Kammer während eines Reinigungszyklus zur Entfernung von Krankheitserregern mittels wenigstens einer, an dem Aufnahmeelement befestigten UV-Lichtquelle UV-C Licht im nicht sichtbaren Wellenlängenbereich und Ozon erzeugt wird und dass die Kammer 2 mit einem Unterdruck und/oder einem Überdruck beaufschlagt wird, wobei mit einem von dem Gehäuse in die Kammer führenden Lufteinlass und einem von der Kammer aus dem Gehäuse führenden Luftauslass ein Luftstrom durch das hohle Aufnahmeelement erzeugt wird und die Luft über Ausgänge an dem hohl ausgebildeten Aufnahmeelement strömt und an Ozon erzeugenden UV-Lampen vorbeiströmt und in den Gegenstand geleitet wird.

Weiterhin kann die Temperatur in der Kammer in einem Temperaturbereich von -50 °C bis 100 °C, einstellbar sein.

Mittels eines von dem Gehäuse in die Kammer führenden Lufteinlasses und eines von der Kammer aus dem Gehäuse führenden Luftauslasses wird dabei ein Luftstrom innerhalb der Kammer erzeugt, der gezielt über in dem Aufnahmeelement angeordnete Kanäle über die UV-Lampe strömt und an bzw. in den zu reinigenden, insbesondere zu desinfizierenden Gegenstand geleitet wird.

Dabei werden bevorzugt Gegenstände gereinigt und desinfiziert, die aus textilem oder polymerem Material bestehen oder die textiles Material aufweisen, welches auch mit anderen Materialien wie Kunststoff, Leder usw. kombiniert sein kann.

Die Kombination von UV-Licht und Ozon, die Temperatur bzw. ein Temperaturwechsel und der Druck bzw. der Druckwechsel in der Kammer gewährleisten ein gutes Reinigungsergebnis und reduzieren bzw. beseitigen die Kontaminierung mit Bakterien, Viren und Keime/Mikroorganismen.

Bevorzugt werden die verwendeten vorgenannten Techniken während eines Reinigungszyklus gleichzeitig durchgeführt. Es ist aber auch möglich, die ausgewählten Techniken nacheinander in beliebiger Reihenfolge bzw. auch alternierend durchzuführen.

Als Mittel zur Ozonerzeugung wird bevorzugt eine UV-C Lampe eingesetzt, mit welcher durch die Abstrahlung von UV-C Licht Ozon erzeugt wird, denn durch die UV-C-Strahlung werden Ionen gebildet, die eine Umwandlung von Luftsauerstoff in Ozon bewirken. Das Ozon ist äußerst reaktiv und wirkt stark keimtötend.

Die UV-C Lampe ist vorzugsweise dimmbar (beispielsweise durch einen vorgeschalteten oder integrierten Dimmer) sodass die Strahlungsintensität der UV-C Lampe in Abhängigkeit vom Verschmutzungsgrad der zu reinigenden Gegenstände verändert werden kann. Bei einer hohen Verschmutzung wird eine hohe Intensität der UV-C Lampe und damit mehr Ozon erzeugt, bei einer geringeren Verschmutzung wird bevorzugt eine niedrigere Intensität der UV-C Lampe und somit weniger Ozon erzeugt. Die Wellenlänge der UV-C Lampen beträgt zwischen 100 nm und 800 nm, bevorzugt zwischen 100 nm und 300 nm, besonders bevorzugt zwischen 150 nm und 280 nm.

Die Temperaturmodi der Vorrichtung sind insbesondere derart einstellbar oder veränderbar, dass die Temperatur in der Kammer von einer niedrigen Temperatur auf eine höhere Temperatur ansteigt, oder von einer hohen Temperatur auf eine niedrigere Temperatur reduziert wird oder dass die Temperatur alternierend ansteigt und wieder absinkt oder absinkt und wieder ansteigt. Die Temperatur kann dabei in einem Temperaturbereich von -50 °C bis 100 °C, bevorzugt in einem Temperaturbereich von -30°C bis 60°C, besonders bevorzugt in einem Temperaturbereich zwischen -10 °C und 50 °C einstellbar und/oder veränderbar sein.

Durch die Temperaturunterschiede werden insbesondere hartnäckige Anhaftungen gelockert, so dass diese leichter gelöst werden können.

Weiterhin kann in der Kammer in Bezug auf den atmosphärischen Druck ein Unterdruck und/oder ein Überdruck erzeugt werden. Der Druck in der Kammer kann dabei von einem niedrigen Druck auf einen höheren Druck ansteigen oder von einem hohen Druck auf einen niedrigen Druck reduziert werden. Auch ist es möglich, dass der Druck in der Kammer alternierend ansteigt und wieder absinkt oder absinkt und wieder ansteigt.

Der Druck in der Kammer kann dabei in einem Druckbereich zwischen 0,001 bar bis 10 bar, insbesondere zwischen 0,1 bar und 2 bar einstellbar und/oder veränderbar sein. Zum Erreichen eines derartigen Druckunterschiedes kann die Kammer innerhalb des Gehäuses luftdicht abgeschlossen sein.

Durch die Druckunterschiede werden Schmutz und andere Anhaftungen von dem zu reinigenden Gegenstand gelöst.

Bei einem Unterdruck wird dabei die Luft aus der Kammer abgesaugt, so dass dadurch Krankheitserreger, Schmutzpartikel und dergleichen aus der Kammer und aus/von den Gegenständen (z.B. von Schuhen) gelöst und abgesaugt werden.

Es ist weiterhin möglich, dass aus einem Reservoir in die Kammer Silber-Nanopartikel eingebracht werden, diese können eine Partikelgröße von 1nm bis 1000nm aufweisen.

Es ist möglich, die Silber-Nanopartikel erst nach Abschluss der anderen Techniken in die Kammer einzubringen, so dass sich diese über die Oberfläche des Gegenstandes legen und dadurch einen zukünftigen Schutz bieten, da es antibakterielle Eigenschaften hat, bzw. eine gute bakterizide Aktivität besitzt.

Vorteilhafter Weise wird in einem Reinigungszyklus erwärmte oder gekühlte Luft in die Kammer eingebracht und in der Kammer mit ultraviolettem Licht aus mindestens einer UV-C Lichtquelle bestrahlt. Mittels der UV-C Lichtquelle wird Ozon in der Kammer erzeugt und dadurch eine Desinfektion und Reinigung des wenigstens eines Gegenstands realisiert, wobei die UV-C Lichtquelle in die Ausnehmungen oder den Hohlraum des Gegenstandes zumindest teilweise eingebracht wird und an dieser ein Luftstrom vorbeiströmt. In der Kammer wird mittels einer Absaugvorrichtung ein bevorzugt alternierender Druck erzeugt, wodurch Verschmutzungen des Gegenstandes gelöst werden und ein Abtransport der Schmutzpartikel erfolgt.

In einer vorteilhaften Ausgestaltung kann der Luftstrom mittels eines Heizelements im Bereich der Ansaugung erwärmt werden. Das Heizelement ist vorzugsweise in Form eines Heizlüfters in den Kanal integriert.

Es ist möglich, dass sich während eines Reinigungszyklus Temperatur vorzugsweise alternierend innerhalb von 0,5 bis 66 Minuten von 150°C auf -30°C reduziert und anschließend wieder erhöht oder umgekehrt..

Weiterhin kann sich während eines Reinigungszyklus der Druck vorzugsweise alternierend innerhalb von0,5 bis 66 Minuten von 5 bar auf 0,1 bar reduzieren und anschließend wieder erhöhen.

Alternativ kann sich während eines Reinigungszyklus die Temperatur nur reduzieren oder nur erhöhen und/oder der Druck sich nur erhöhen oder nur reduzieren.

Besonders bevorzugt wird dem Luftstrom im Bereich der Zuführung in die Kammer Wasserdampf zugemischt, wodurch eine glättende Wirkung auf die Textilien ausgeübt wird. Das Bügeln der Kleidungsstücke kann dann entfallen.

Der Wasserdampf wird in einem Wassertank mit integriertem Heizelement erzeugt. Durch die Verwendung von heißem Dampf mit einer Temperatur von 65 bis 150°C werden ebenfalls am Kleidungsstück oder einem medizinischen Gegenstand vorhandene Bakterien, Pilze und Viren abgetötet.

Vorzugsweise wird dem Luftstrom und/oder der Kammer ein Duft zugefügt. Die Duftelemente können austauschbar und in verschiedenen Düften verfügbar sein.

Insgesamt ist es mit dem Verfahren möglich, Bakterien, Viren, Pilzen und deren Sporen in bzw. an Gegenständen abzutöten.

Insbesondere Schutzausrüstung und Schutzkleidung sowie Laborgegenstände aus typischen textil- oder chemischen Polymeren und/oder Polymerfasern können in kurzer Zeit, insbesondere innerhalb von 1 bis 100 Minuten und bevorzugt innerhalb von 1 bis 15 Minuten gereinigt und desinfiziert werden.

Dies erfolgt schnell und schonend, wobei die Gegenstände nach Beendigung des Reinigungsvorganges sofort wiederverwendbar sind.

Beispielsweise Schutzkleidung wie Mundschutz, Schutzkittel, Handschuhe, Schuhe und dergleichen, die nur für den einmaligen Gebrauch bestimmt waren, können dadurch bedenkenlos nochmals bzw. auch mehrmals verwendet werden, da vorhandene Krankheitserreger gemäß Tabelle 1 eliminiert worden sind.

Insgesamt wird mit der erfindungsgemäßen Lösung eine äußerst zuverlässige Reinigung und Desinfektion des/der Gegenstandes/Gegenstände erzielt. Insbesondere können in dem Gegenstand oder Kleidung vorhandene Ausnehmungen und Hohlräume gereinigt und desinfiziert werden, wenn man die UV-Lampe in diese zumindest teilweise einbringt. Dadurch, dass auch in den Hohlräumen die Druckunterschiede wirken und die Temperatur in der Kammer gekühlt oder erwärmt wird (ggf. auch alternierend) werden auch Hohlräume und Ausnehmungen von Gegenständen gemäß Tab. 2, z.B. wie bei Kleidungsstücken (z.B. das Innere von Schuhen oder Handschuhen), oder auch von Kitteln und Jacken und anderen Textilien, die insbesondere im medizinischen und Laborbereich Anwendung finden, gereinigt und desinfiziert. Dadurch ist eine zuverlässige und nahezu vollständige Dekontamination der Gegenstände von in Tabelle 1 genannten Krankheitserregern möglich.

Besonders vorteilhaft ist es das erfindungsgemäße Verfahren anzuwenden, wenn Gegenstände aus textilem Material bestehen oder dieses aufweisen und nicht oder nur eingeschränkt gewaschen werden können bzw. keine Wasserversorgung zum Waschen zur Verfügung steht. Wenn Gegenstände nicht oder nur leicht verschmutzt sind und daher ein Waschen mit Wasser nicht unbedingt erforderlich ist, ist ebenso eine Nutzung des erfindungsgemäßen Verfahrens zur Entfernung bzw. Desinfektion von Bakterien, Viren, Pilze u.a. Pathogenen angezeigt.

Die Vorrichtung zur Reinigung und Desinfizierung wenigstens eines Gegenstandes, insbesondere eines Kleidungsstückes oder von wenigstens einer Ausnehmung oder Hohlraum in einem Gegenstand, weist eine von einem Gehäuse umgebene Kammer auf, in welche der Gegenstand an einem Grundkörper aufgehangen ist, wobei die Vorrichtung erfindungsgemäß die nachfolgenden Mittel A) bis D) aufweist:
A) wenigstens eine in der Kammer an dem Aufnahmeelement angeordnete UV-Lampe zur Erzeugung von UV-C Licht im nicht sichtbaren Wellenlängenbereich,
B) wenigstens ein Mittel zur Ozonerzeugung,
C) wenigstens eine Einrichtung zum Einstellen und/oder Verändern des Drucks in der Kammer
D) wenigstens eine Einrichtung zur Einstellung und/oder Veränderung der Temperatur in der Kammer,

Des Weiteren weist die Vorrichtung eine von außerhalb des Gehäuses in das Innere der Kammer weisende Luftzuführung mit einem Lufteinlass und mit wenigstens einem, sich entlang der Luftzuführung erstreckenden Heizelement und einen von der Kammer aus dem Gehäuse weisenden Luftauslass auf.

Vorzugsweise ist an dem Lufteinlass und dem Luftauslass ein Lüfter angeordnet, der Luft anbeziehungsweise absaugt.

Die Vorrichtung weist alle vorgenannten Mittel auf, wobei mit diesen je nach Bedarf mehrere oder alle der damit durchführbaren Techniken während eines Reinigungszyklus realisiert werden, auch in voreingestellten Zyklen.

An dem in der Kammer Grundkörper ist vorteilhafter Weise wenigstens eine UV-Lampe zur Erzeugung von (nicht sichtbarer) UV-C Strahlung und zur Erzeugung von Ozon angeordnet. Weiterhin weist die Vorrichtung die Absaugeinrichtung zur Erzeugung eines Unterdrucks in der Kammer auf. Mit dieser Absaugeinrichtung werden auch die vom Gegenstand gelösten Schmutzpartikel abgesaugt.

Vorteilhafter Weise werden wenigstens zwei UV-Lampen an dem Grundkörper nebeneinander und zueinander beabstandet angeordnet, wobei die UV-Lampen insbesondere dimmbar sind.

Der Grundkörper kann in einer vorteilhaften Ausgestaltung in seiner Breite und/oder Höhe verstellbar ausgebildet sein. Dies erfolgt vorzugsweise mittels nach außen bewegbarer bzw. schwenkbarer Elemente, die stufenlos oder mittels Rasterung verstellbar sind.

Der Grundkörper ist entnehmbar und/oder austauschbar in der Kammer befestigt oder kann beispielsweise mittels eines Schienensystems aus der Kammer ausfahrbar und in die Kammer einfahrbar sein. So kann der Gegenstand bequem außerhalb der Kammer auf dem Grundkörper fixiert und anschließend in die Kammer eingebracht werden.

Der Reinigungszyklus ist vorteilhafter Weise mittels einer Steuereinheit einstellbar und die Parameter wie Temperatur und/oder Druck und/oder Zugabe von Duftstoffen und/oder Silber-Nanopartikeln einstellbar sind.

In einer vorteilhaften Ausgestaltung ist in der Luftzuführung ein Duftelement angeordnet. Das Duftelement kann einstellbar sein, derart dass die gewünschte Intensität des Duftes der Kleidung oder des Textils eingestellt werden.

Die Vorrichtung weist vorzugsweise einen Wassertank mit einem Heizelement auf derart, dass Wasserdampf erzeugbar ist und dass der Wasserdampf über einen Kanal der Luftzuführung zuführbar ist. So wird ein kombinierter Luftstrom aus erwärmter Luft und Wasserdampf in die Kammer (Reinigungskammer) gegeben und in die Kleidung eingebracht.

Das erfindungsgemäße Verfahren dient insbesondere der Reinigung und Desinfizierung von Gegenständen, insbesondere von, von Textilien gebildeten Hohlräumen und textilen Flächengebilden, wobei die Textilien (bzw. wenigstens eine Textilie) durch eine Öffnung in eine, von einem Gehäuse gebildete Kammer eingelegt werden. Bevorzugt wird in einem ersten Verfahrensschritt in die Kammer temperaturveränderliche Luft, vorzugsweise erwärmte oder gekühlte Luft eingebracht und anschließend den Gegenstand/Gegenständen mit ultraviolettem Licht aus wenigstens einer UV-Quelle bestrahlt, wobei mittels der UV-Quelle Ozon in der Kammer erzeugt wird und eine Desinfektion und Reinigung der Gegenstände erfolgt. Der von wenigstens einem Textil gebildete Hohlraum wird auf wenigstens einer UV-Quelle angeordnet derart, dass die UV-Quelle in die Gegenstände zumindest teilweise einbringbar oder nahe an dem Gegenstand positionierbar ist. Die Keime und Bakterien werden durch die Kombination aus UV-Strahlung und dem gebildeten Ozon abgetötet, wodurch eine Desinfektion des betreffenden Gegenstandes umsetzbar ist. Die Reinigungswirkung wird durch die Luftströmung, die Temperatur und den Druck in der Kammer verbessert.

Das erzeugte Ozon wird durch einen im Bereich der Absaugvorrichtung eingebrachten Filter entfernt, wenn die gesäuberte Luft mittels der Absaugvorrichtung aus der Kammer abgesaugt bzw. ausgestoßen wird.

In der Kammer wird mittels der Absaugvorrichtung wechselweise ein Unterdruck bis hin zu einem Vakuum erzeugt, mittels dessen ein Lösen von Verschmutzungen in den Gegenständen und ein Abtransport von gelösten Schmutzpartikeln mittels der Absaugung erfolgen.

Die UV-Quelle arbeitet mit einer Wellenlänge der ultravioletten Strahlung im nicht sichtbaren UV-C Bereich, wobei die Wellenlänge 100 nm und 800 nm, bevorzugt zwischen 100 nm und 300 nm, besonders bevorzugt zwischen 150 nm und 280 nm beträgt. Auch die Nutzung von Infrarot-Strahlung ist denkbar.

Vorzugsweise sind der Kammer mit Duftstoffen angereicherte Luft zuführbar. So kann das zu reinigende Textil bzw. der Gegenstand mit einem angenehmen Geruch versehen werden.

Die Vorrichtung dient der Reinigung von Textilien und med. Schutzausrüstung bzw. biochem. aktives Material, insbesondere von mit Keimen, Bakterien und Pilzen behafteten Gegenständen, wobei die Vorrichtung ein Gehäuse und eine, im Inneren des Gehäuses angeordnete Kammer mit einem Grundkörper zur Aufnahme für Textilien oder Gegenstände aufweist. Die Vorrichtung weist eine, von außerhalb des Gehäuses in das Innere der Kammer weisende Luftzuführung mit wenigstens einem, sich entlang der Luftzuführung erstreckenden Heiz- und/oder Kühlelement auf. Am Aufnahmeelement ist mindestens eine UV-Lampe angeordnet, wobei die UV-Lampe zur Erzeugung von UV-Strahlung vorgesehen ist derart, dass in der Kammer Ozon generierbar ist. Des Weiteren weist die Vorrichtung eine Absaugeinrichtung zur Erzeugung eines Unterdrucks in der Kammer auf, wodurch die Luft und Schmutzpartikel aus der Kammer absaugbar beziehungsweise förderbar sind, wobei das generierte/verwendete Ozon durch einen Filter entfernt wird derart, dass gesäuberte Luft ausgestoßen wird.

Die Absaugeinrichtung ist in einer vorteilhaften Ausgestaltung derart dimensioniert, dass diese ein Vakuum in der Kammer erzeugen kann. Somit lassen sich Schmutz und Keime aus den Textilien besser lösen, insbesondere wenn ein wechselnder Unterdruck erzeugt wird.

Vorzugsweise sind an der Vorrichtung eine Zuführung für Silber-Nanopartikel und/oder Duftstoffe vorgesehen. Die Silber-Nanopartikel setzen sich in den Gegenständen ab und dienen der Vorbeugung von Viren- und Bakterienwachstum in den gereinigten Gegenständen.

Dabei werden die Silber-Nanopartikel und/oder Duftstoffe in die Kammer eingelegt. Bevorzugt werden dann die Silber-Nanopartikel und/oder Duftstoffe direkt in den/die von dem/den Gegenstand/Gegenständen gebildeten Hohlraum/Hohlräumen eingebracht. Dies erfolgt bevorzugt über die Luftzuführung.

Die, in die Kammer einbringbare Luft kann mittels einer Heizspirale, die um die Luftzuführung angeordnet ist, erwärmt oder über eine Kühleinrichtung abgekühlt werden. Am gehäuseseitigen Ende der Luftzuführung ist vorzugsweise ein Lüfter angeordnet, der die Luft ansaugt und in die Kammer befördert. Es kann auch ein kombinierter Heizlüfter Anwendung finden
Die Absaugung erfolgt auf eine ähnliche Weise, wobei ein zweiter Lüfter die Luft aus der Kammer absaugt und in die Umgebung abgibt.

Es ist auch möglich, dass eine Heiz- und/oder Kühlvorrichtung in der Kammer angeordnet ist und die zugeführte Luft nicht erwärmt bzw. Gekühlt wird.

Vorzugsweise ist der Reinigungszyklus mittels einer Steuereinheit einstellbar, wobei einzelne oder alle Parameter anpassbar sind. Die Parameter umfassen die Temperatur und/oder den Druck innerhalb der Kammer sowie, sofern gewünscht, die Zugabe von Duftstoffen und/oder den Silber-Nanopartikeln. Die Kammer kann des Weiteren zum Trocknen der Gegenstände Anwendung finden. Die Vorrichtung nutzt als UV-Quelle das Spektrum des UV-C Lichts, wobei die Kombination aus UV-Strahlung, Ozon, Temperaturprogrammen und Vakuum-Luftwechsel-Einsatz Bakterien, Viren und Pilze eliminieren. Die Parameter können von der Vorrichtung selbstständig über die Eingabe von Eigenschaften des zu reinigenden Gegenstandes ermittelt werden. Diese Eigenschaften umfassen beispielsweise die Art des Materials, Verschmutzungsgrad, Feuchtigkeitsgrad oder ähnliches.

Die einstellbaren Temperaturprogramme umfassen dabei die Möglichkeit von erwärmter oder auch stark abgekühlter Luft, die an die anzuwendenden Reinigungsorte geleitet wird. Das Ozon wird unmittelbar an der UV-C Quelle, der UV-Lampe synthetisiert, d.h. am gewünschten Anwendungsbereich synthetisiert und angewendet.

Das Vakuum-Luft-Wechsel-System entfernt abgestorbene und eventuell noch Organismen aus den Textilien.

Die Kammer wirkt während des Reinigungszyklus antiviral, antibakteriell und antimykotisch und reinigt die Produkte zuverlässig, ausgehend von dem inneren Bereich, in dem sich die UV-Lampen befinden. Die Luft mit dem Ozon durchdringt die Textilien und sorgt dadurch für eine äußerst zuverlässige Reinigung und Desinfektion. Die Desinfizierung und Reinigung erfolgt ohne den Zusatz von Chemie, wodurch eine hohe Umweltfreundlichkeit und Materialschonung erzielt werden. Durch die Nutzung von Wasserdampf ist auch nur eine geringe Menge an Wasser für die Reinigung bzw. Glättung der Gegenstände notwendig.

Vorzugsweise werden mit den Gegenständen, wie z.B. Jacken, Kittel, Handschuhe, Schuhe, Mundschutz für den medizinischen und Laborbereich gereinigt. Es ist jedoch auch möglich, andere Gegenstände für medizinische und Laboreinrichtungen zuverlässig zu desinfizieren.

Die Vorrichtung und die darin angeordnete Kammer sind derart ausgebildet, dass Gegenstände problemlos und knitterfrei hineinpassen und darin angeordnet oder aufgehangen werden können. Die Abmessungen in Bezug auf Breite, Höhe und Tiefe betragen bevorzugt 30/125/60cm,

Eine Vergrößerung der Abmessungen, z.B. auf ein Apparatevolumen von 2 bis 5 m³ mit entsprechend mehr UV-C bzw. Ozon-Quellen mit größerer Pump- und Heizleistung bzw. größeren Bauelementen hat keinen Einfluss auf die Effektivität der Wirkungsweise. Bevorzugt soll die Vorrichtung die genannten Maße nicht unterschreiten.

Die erfindungsgemäße Vorrichtung wirkt gegen praktisch alle Pathogene, wie Viren und Bakterien sowie verschiedene Milbenarten wie Hausmilben und Krätze-Milben sowie gegen Pilze in Form von Fußpilz oder Hautpilz gemäß Tab. 1

Weiterhin werden durch Bestrahlung der Gegenstände und Kleidung mit ultraviolettem Licht und/oder Anwendung von Ozon Organismen inaktiviert oder zerstört. Dies kann als Desinfektion des bestrahlten Bereichs angesehen werden. Diese mikroskopischen Organismen umfassen beispielsweise sporenbildende und nicht-sporenbildende Lebensformen oder Viren oder Bakteriophagen oder Zysten gemäß Tab. 1

Die erfindungsgemäße Vorrichtung findet insbesondere Anwendung zur Desinfizierung/Abtötung/Keimeliminierung von Viren, speziell Covid-19 Corona Viren bzw. Viren der SARS Familie und der in Tabelle 1 aufgelisteten Mikroorganismen, insbesondere für Arbeitsbekleidungen oder auch Einmalbekleidung in Laboren und medizinischen Bereich.

Dazu wird in der Vorrichtung UV-Licht mit einem Wellenlängenbereich von 185 nm bis 400 nm, Ozon, eine Temperatur von 20-100°C, auch wechselnd, ein (Unter-)Druck von 0,1bar bis 5bar erzeugt und damit die Gegenstände (z.B. die Kleidung) beaufschlagt.

Es ist somit möglich, dass in den Grundkörper über eine entsprechende Pumpe Luft mit Druck eingeblasen wird, die dann durch den Grundkörper über die Durchströmöffnungen an den UV-C Lampen vorbei gegen in bzw. n die zu reinigenden Gegenstände, vorzugsweise Textilien, strömt und dann über einen Filter und einen Luftauslass in die Umgebungsluft abgeführt wird.

Es können weitere Durchströmöffnungen (ohne UV-C Lampen) in dem Grundkörper angeordnet sein, durch welche Luft gegen die zu reinigenden Gegenstände ausströmt. Dann wird, wenn erwünscht, auch zusätzlich ein Trocknungsprozess erzielt.

Weiterhin ist es möglich, dass Luft aus dem Hohlraum des Grundkörpers über eine entsprechende Pumpe/Vakuumpumpe abgesaugt wird, die Strömungsrichtung ist dann entgegen gesetzt. Ist die Pumpe an den Grundkörper angeschlossen und wird aus diesem Luft abgesaugt (und ein Vakuum erzeugt), wird die Luft aus der Kammer über die auf dem Grundkörper aufgenommenen Gegenstände und die UV-C Lampen in den Hohlraum des Grundkörpers gesaugt und dann nach außen gefördert. Dadurch werden in der Vorrichtung und an den zu reinigenden Gegenständen befindliche Partikel (z.B. Schmutz, Krankheitserreger, abgetötete) abgesaugt.

Überdruck und der Unterdruck können durch eine oder zwei getrennte Pumpen erzeugt werden.

Des Weiteren ist die Vorrichtung in einer vorteilhaften Ausgestaltung auch zur mobilen Anwendung geeignet.

Es ist ebenfalls möglich, auch Wasserdampf oder Lösungsmitteldämpfe, flüssiges oder gasförmiges Fluid - welches Lösungsmittel enthalten, durch die Grundkörper strömen zu lassen, wodurch beispielsweise Flecken, Verschmutzungen bzw. Verunreinigungen entfernt werden können. Weiterhin kann Wasserdampf mit Überdruck und/oder Unterdruck durch die Grundkörper und über die Durchströmöffnungen zu den zu reinigenden Gegenständen zu führen.

Auch der Wasserdampf kann ggf. mit einem Lösungsmittel gegen Flecken vermischt sein. Unterdruck können Flecken oder andere Verunreinigungen effektiv entfernt werden über- und Unterdruck.

Durch das Hindurchströmen von Wasserdampf, Lösungsmitteldämpfen, und ggf. anderen geeigneten Fluids können, bevorzugt mit wechselndem Druck, insbesondere Unter- und Überdruck können Flecke und andere Verunreinigungen effektiv entfernt werden.

Dadurch, dass mittels der Luftzuführung erwärmte/gekühlte Luft in den vorgenannten Temperaturbereichen in die Kammer geleitet wird, bzw. die Luft in der Kammer erwärmt oder gekühlt wird, dass die UVC-Strahlungsquellen in die Gegenständen eingebracht sind, bzw. nahe an diesen positioniert sind ist eine bessere Durchdringung der Gegenständen mit dem Ozon über den unmittelbar in die Gegenständen eingeleiteten und darauf wirkenden Luftstrom gewährleistet, als bei dem Stand der Technik und es wird dadurch eine bessere Reinigungswirkung bzw. Desinfektionswirkung und Entkeimung von Bakterien und Viren erzielt.

Diese Wirkung wird noch weiter verbessert, wenn die Grundkörper für die zu reinigenden Gegenstände mit einem kammerseitigen Ende der Luftzuführung verbunden sind, und eine Aufteilung des Luftstroms innerhalb der Kammer mit dem Grundkörper erfolgt, durch welchen der Luftstrom geleitet wird.

Der hohl ausgeführte Grundkörper kann beispielsweise aus Rohren, Rohrabschnitten und/oder Rohrverbindungselementen bestehen bzw. daraus zusammengesetzt sein. Die Vorsprünge, die sich an den hohlen, beispielsweise rohrförmigen Grundkörper anschließen und/oder die zusätzlichen Öffnungen können dabei beispielsweise aus Rohrfittingen, Rohrbögen, Kreuzstücken, Rohrnippeln und dergleichen gebildet werden.

Die UV-Lampen weisen bevorzugt ein Außengewinde auf, welches mit dem Innengewinde der Rohre, Rohrbögen, Kreuzstücke, Rohrnippel korrespondieren.

Der rohrförmige Grundkörper kann mit den Rohrbögen, Kreuzstücken, Rohrnippeln und dergleichen verschraubt, verklebt, mittels Pressverbindern verbunden werden.

Der rohrförmige Grundkörper sowie die Verschraubungselemente und Rohrverbindungselemente und dergleichen bestehen beispielsweise aus Rotguss, Messing, Edelstahl oder Temperguss oder können auch aus Kunststoff gefertigt sein.

Dass der rohrförmige Grundkörper und die Verschraubungselemente bzw. die Durchströmöffnungen oder Vorsprünge in einem Grundkörper wiesen ein Anschlussgewinde von 3/4 Zoll bis 1 1/4 Zoll auf.

Bevorzugt ist das Gewinde für das Einschrauben der UVC-Leichtmittel ein 1 ¼ Zoll Innengewinde. Das Gewinde gängiger UVC-Lampen passt formgenau in die 1 1/4 Zoll Gewinde.

Dabei weisen die Rohre, Rohrverbindungselemente, Anschlussstücke und dergleichen bevorzugt einen Außendurchmesser von 32mm auf.

Alternativ besteht der hohle Grundkörper auch nur aus einem einzelnen Stück und ist mittels 3D-Druck gefertigt oder beispielsweise durch Spritzgießen.

Zusätzlich zu den Öffnungen im Grundkörper im Bereich der UV-Lampen können weitere Durchströmöffnungen vorhanden sein.

Die Vorrichtung findet somit zum Abtöten von Bakterien, Viren und Pilzen u.a. Pathogenen auf oder in Gegenständen, insbesondere Kleidungsstücken bzw. Schutzkleidung, Schutzausrüstung, für den medizinischen, veterinärmedizinischen oder Laborbereich und besonders bevorzugt Gegenstände im medizinischen, veterinärmedizinischen oder Laborbereich Anwendung.

Es kann selbstverständlich auch die Berufsbekleidung von anderen Berufsgruppen gereinigt werden, z.B. von Pflegeberufen oder Berufen, die in engen Kontakt mit anderen mit anderen Personen gelangen. Aber auch für das Hotels zur Reinigung der Kleidung des Personals oder der Gäste oder auch im privaten Bereich, z.B. wenn sich gefährdete Personen sich Haushalt befinden, kann die Vorrichtung zur Reinigung und Desinfektion von Kleidung und/oder anderen Gegenständen verwendet werden.

Vorteilhafter Weise besitzen die Gegenstände, insbesondere medizinische Geräte und Gegenstände und medizinische Schutzbekleidung an ihren Oberflächen am Ende jeweils einer Behandlung nur noch Reste von >kleiner = 10%, bevorzugt kleiner gleich 1%, insbesondere kleiner gleich 0,1% an biochemisch biologisch- aktiver Organismen und/oder biochemischer Materialien.

Die Gegenstände, die normaler Weise für nur für einen einmaligen Gebrauch bestimmt sind (Einweg-Atemmasken, Schutzhandschuhe, Schutzkittel und dergleichen) sind dadurch nach einer Behandlung wieder verwendbar.

Die Erfindung wird nachfolgend an einem Ausführungsbeispiel und zugehörigen Zeichnungen näher erläutert. Es zeigen:
- Figur 1: eine Vorrichtung gem. Figur 2 und 3 in geschlossener Position,
- Figur 2: eine Seitenansicht einer Vorrichtung mit einem ersten und zweiten Grundelement innerhalb der Kammer,
- Figur 3: eine dreidimensionale Darstellung gemäß Figur 2,
- Figur4: eine Darstellung, ähnlich wie Figur 6 jedoch ohne Deckel in dem Einschub und dafür mit Querstreben 12 zwischen den Seiten S1 und S2,
- Figur 5: einen weiteren Aufbau einer erfindungsgemäßen Vorrichtung in 3D-Ansicht
- Figur 6: in der Vorderansicht und
- Figur 7: in der Seitenansicht.
- Figur 8: Vorderansicht eines Schuh-Adapters,
- Figur 9: 3D-Ansicht eines Schuhadapters,
- Figur 10: Vorderansicht eines Kleidungs-Adapters,
- Figur 11: 3D-Ansicht eines Kleidungs-Adapters,
- Figur 12: zwei miteinander verbindbare Halbschalen,
- Figur 13: eine Halbschale, die mit einer Platte verschlossen wird,
- Figur 14: Vorrichtung in Form einer Box mit in Längsrichtung eingeschobenem Adapter,
- Figur 15: die Box mit herausgezogenem Adapter,
- Figur 16: eine Box mit quer in der Kammer aufgenommenem Adapter,
- Figur 17: runder drehbarer Grundkörper mir einer Vielzahl von Vorsprüngen in der Seitenansicht,
- Figur 18: runder Grundkörper gem. Figur 20 in der Draufsicht,
- Figur 19: der Grundkörper gemäß Figur 20 in einer Box RB,
- Figur 20: eine Vorrichtung mit mehreren rohrförmigen Grundkörpern, die sich vertikal nebeneinander erstrecken und untereinander verbunden sind,
- Figur 21: die Vorderansicht gemäß Figur 20,
- Figur 22: die Vorrichtung gemäß Figur 20 und 21 aus einer anderen Perspektive mit einem beispielhaft über einem Grundkörper positionierten Laborkittel,
- Figur 23: eine Vorrichtung mit rohrförmigen Grundkörpern, bei welcher eine Vielzahl von sich nach oben erstreckenden Vorsprüngen neben- und übereinander angeordnet sind,
- Figur 24: die Vorderansicht gemäß Figur 23,
- Figur 25: eine Vorrichtung mit einem kreuzförmigen hohlen Grundkörper mit drei nach oben weisenden Vorsprüngen,
- Figur 26: eine Teilansicht der Vorrichtung gemäß Figur 25 mit darüber aufgenommenen zu reinigenden Gegenständen,
- Figur 27: einen Grundkörper, der sich im Wesentlichen horizontal erstreckt mit drei nach oben weisenden Durchströmöffnungen,

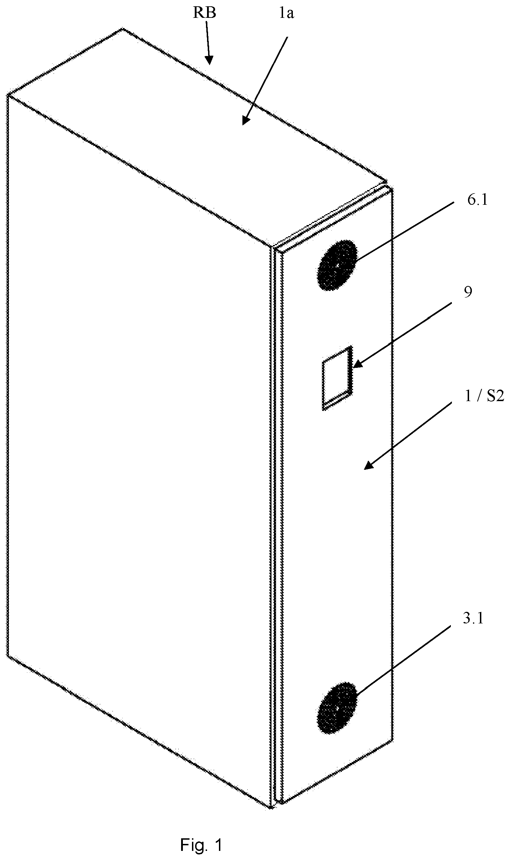

In den Figuren 1 bis 3 ist ein schematischer Aufbau der Vorrichtung zum Reinigen und Desinfizieren von Gegenständen, insbesondere zum Reinigen und Desinfizieren des Inneren von Gegenständen, wie z.B. medizinischen Ausrüstungen, Schuhen, Helmen, Handschuhe, Sportequipment, T-Shirts, Hemden, Anzügen (Kleidungsstücken) und Taschen dargestellt. Die Vorrichtung umfasst eine Box RB mit einer Kammer 2/Reinigungskammer, die hier in einem Gehäuse 1 ausgebildet ist, wobei das Gehäuse 1 über ein nicht dargestelltes Schienensystem in einen, das Gehäuse 1 umschließenden Schrankkorpus 1a der Box RB einschiebbar und herausziehbar ist. Der Schrankkorpus 1a ist in Richtung zum Gehäuse 1 offen (über eine sich vertikal erstreckende nicht bezeichnete Öffnung), so dass das Gehäuse 1 in diesen einschiebbar ist. Ansonsten ist der Schrankkorpus unten, oben, an den Seiten und der Rückseite geschossen. Das in den Schrankkorpus 1a einschiebbare Gehäuse 1 mit der Kammer 2 (Reinigungskammer) ist hier bevorzugt rahmenartig ausgebildet. Das Gehäuse 1 weist einen Boden B und einen Deckel D auf, wobei der Boden B und der Deckel D über zwei endseitige vertikale und zueinander parallele Seitenteile S1, S2 miteinander verbunden sind. Das Seitenteil S1 ist in Richtung zum Schrankkorpus 1a angeordnet und das Seitenteil S2 dazu entgegengesetzt. Das Seitenteil S2 verschließt im eingeschobenen Zustand den Schrankkorpus 1a. (Siehe Figur 1)

Die Vorrichtung weist im unteren Bereich eine Luftzuführung 3 in Form eines Kanals auf, wobei das Gehäuse 1 an seiner Front eine Öffnung in Form eines Lufteinlasses 3.1 für die in die Kammer 2 führende Luftzuführung 3 aufweist, wobei an dem gehäuseseitigen Ende der Luftzuführung 3, welches den Lufteinlass 3.1 bildet, ein Lüfter 3.2 angeordnet ist. Der Lüfter 3.2 ist derart ausgebildet, dass frische Luft aus der Umgebung angesaugt und durch die Luftzuführung 3 in die Kammer 2 förderbar ist.

Die Luftzuführung 3 ist vorzugsweise rohrförmig ausgebildet und weist an ihrem kammerseitigen Ende 3.3 einen Winkel auf, vorzugsweise von 90°. Der Winkel wirkt als Führung der Luft, wodurch eine zentrale Ausströmung der angesaugten Luft in der Kammer 2 erreichbar ist.

Es sind zwei hohl ausgebildete Grundkörper 4 vorgesehen, die beide mit dem kammerseitigen Ende der Luftzuführung verbunden sind und sich nach oben in die Kammer 2 erstrecken.

Jeder Grundkörper 4 weist eine Luftführung auf, mittels derer der durch das kammerseitige Ende geführte Luftstrom in einen ersten und zweiten Luftstrom aufteilbar ist. Je Luftstrom weist der jeder Grundkörper eine, an einer Austrittsöffnung des Luftstroms angeordnete UV-Strahlungsquelle auf.

Die Austrittsöffnungen sind in Abhängigkeit der Ausgestaltung des Grundkörpers radial oder im dargestellten Beispiel axial in Bezug auf die Strömung der Luft angeordnet.

Die Wellenlänge der UV-Strahlung beträgt vorzugsweise zwischen 100 und 300 nm. Die ultraviolette Strahlung ist derart kurzwellig und energiereich, dass sie durch molekularen Sauerstoff (O₂) absorbiert wird. Dabei wird der molekulare Sauerstoff (O₂) in zwei freie Sauerstoffradikale (2 O^{·}) gespalten, die jeweils mit einem weiteren Molekül Sauerstoff (O₂) zu Ozon (O₃) weiterreagieren.

Das derart gebildete Ozon an der UV-Lampe 5 desinfiziert die Gegenstände gegen Keime, Pilze und Bakterien.

Des Weiteren weist die Kammer 2 (Hauptkammer bzw. Reinigungskammer) eine Absaugeinrichtung 6 im Deckenbereich der Kammer 2 auf. Die Absaugeinrichtung 6 ist bevorzugt im oberen Bereich der Vorrichtung angeordnet, hier oben im Seitenteil S2, und weist beispielsweise einen durch das Gehäuse 1 an dessen Front nach außen führenden Luftauslass 6.1 auf, in dem ein zweiter Lüfter 6.2 montiert ist. Der Lüfter 6.2 ist derart ausgebildet, dass die Luft aus der Kammer 2 gesaugt wird, wodurch ein Unterdruck entsteht. Es wird ein wechselnder Druck in der Kammer 2 erreicht, wobei die Absaugeinrichtung 6 derart ausgebildet sein kann, dass ebenfalls ein Vakuum in der Kammer 2 erzeugbar ist. Das Vakuum-Luft-Wechsel-System entfernt abgestorbene, lebende und eliminierte Organismen aus den Gegenständen. Der Absaugeinrichtung 6 mit dem Luftauslass 6.1 kann ein nicht dargestellter austauschbarer Filter vorgeschaltet sein, sodass die verschmutzte Luft vor Austritt aus der Kammer gereinigt und das Ozon entfernt werden kann.

Für eine effektivere Reinigung wird die Luft im Bereich der Luftzuführung 3 erwärmt oder abgekühlt oder auch abwechselnd erwärmt und gekühlt. Für diesen Verfahrensschritt ist im Bereich der Luftzuführung 3 eine Heizeinrichtung 3.4 angeordnet, die vorzugsweise in Form einer Heizspirale um die Luftzuführung 3 gewickelt ist. Alternativ kann auch ein Heizlüfter als Kombination aus Heizeinrichtung 3.4 und Lüfter 3.2 eingesetzt werden. Die durch die Luftzuführung 3 strömende Luft wird im Bereich der Heizeinrichtung 3.4 erwärmt. Alternativ oder zusätzlich kann in dem Bereich der Luftzuführung 3 ein Kühlelement 6.1 vorgesehen sein.

Es ist auch möglich, ein Heiz, bzw. Kühlelement in der Reinigungskammer 2 und entsprechend den Innenraum der Kammer zu erwärmen bzw. abzukühlen.

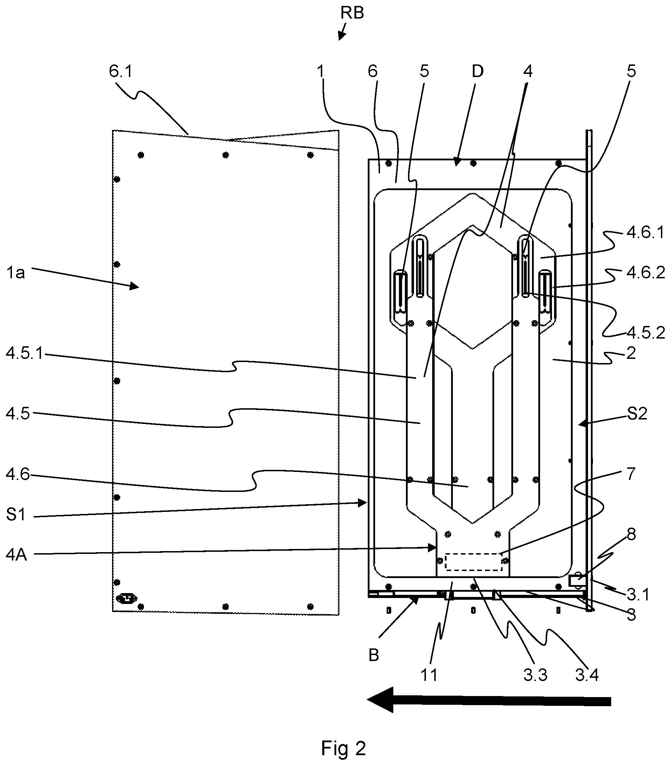

In einer vorteilhaften Ausgestaltung ist gemäß den Figuren 1 und 2 im Bodenbereich der Vorrichtung ein Wassertank 7 mit einem in diesem angeordneten Heizelement 7.1 vorgesehen. Das Wasser wird derart erwärmt, dass Wasserdampf entsteht, wobei der Wasserdampf über eine Leitung 7.2 der Luftzuführung 3 zugeführt wird.

Es erfolgt eine Vermischung von erwärmter Luft und dem erzeugten Wasserdampf, wobei das Gemisch über das kammerseitige Ende 3.3 der Kammer 2 zugeführt wird.

Durch die mit Feuchte angereicherte Luft erfolgt eine Glättung des zu reinigenden Kleidungsstückes, wodurch ein späteres Bügeln entfällt. Der Wassertank 7 ist vorteilhafter Weise über eine seitliche Öffnung befüllbar oder komplett entnehmbar ausgebildet.

Es ist auch möglich, dass der Boden B doppelwandig ausgebildet ist und durch den dadurch gebildeten bodenseitigen Hohlraum die Luft von dem Lufteinlass 3.3 über die Luftzuführung 3 in die Kammer 2 geleitet wird. Analog kann durch den Bodenseitigen Hohlraum auch der Wasserdampf über den Lufteinlass 3 oder einen weiteren Einlass (nicht dargestellt) in die Kammer 2 strömen.

Im dem Bodenseitigen Hohlraum sind dann auch der Lüfter 3.2, die Heizeinrichtung 3.4, der Wassertank 7 und das Heizelement 7.1 angeordnet.

Zusätzlich kann zur Erzeugung eines Frischeduftes der Gegenstände eine Duftkapsel 8 in die Luftzuführung 3 einlegbar sein. Die freigesetzten Duftstoffe können mit der von der Luftzuführung 3 angesaugten Luft zugesetzt und in die Kammer 2 geleitet werden. Vorzugsweise ist die Duftintensität über einen manuellen Regler 8.1 oder über die Steuerung 9 mit einem frontseitigen Display der Vorrichtung einstellbar.

Die Vorrichtung bietet zudem die Möglichkeit feuchte Gegenstände in kurzer Zeit schonend zu trocknen.

Mit der erfindungsgemäßen Vorrichtung ist eine umweltschonende Reinigung und Desinfektion der Gegenstände ohne die Zugabe von chemischen Reinigungsmitteln möglich. Viren, Bakterien, Keime und Pilze und deren Sporen gemäß Tab. 1 und andere Pathogene werden hierbei effektiv beseitigt.

In Figur 1 ist die Vorrichtung im geschlossenen Zustand dargestellt. Der Einschub in Form des Gehäuses 1 ist in den Schrankkorpus 1a der Box RB eingeschoben, wobei das Seitenteil S2 den Schrankkorpus 1a abschließt. Zwischen dem Seitenteil 1 und dem Schrankkorpus 1a kann dabei eine Dichtung vorgesehen sein.

In dem Seitenteil S2 befinden sich das Display 9, der Lufteinlass 3.1 und der Luftauslass 6.1. Alternativ können sich der Lufteinlass 3.1 und der Luftauslass auch in dem Rückseitigen Seitenteil S1 befinden (nicht dargestellt), wobei dann der Schrankkorpus 1a an seiner nicht bezeichneten Rückseite zumindest in diesen Bereichen offen sein sollte.

Die Bedienung der Vorrichtung erfolgt mittels des Displays 9 oder auch über eine App.

Es ist möglich, in der Kammer 2 einen oder mehrere Grundkörperanzuordnen, so dass ein oder mehrere Gegenstände gleichzeitig desinfiziert und aufgefrischt werden können.

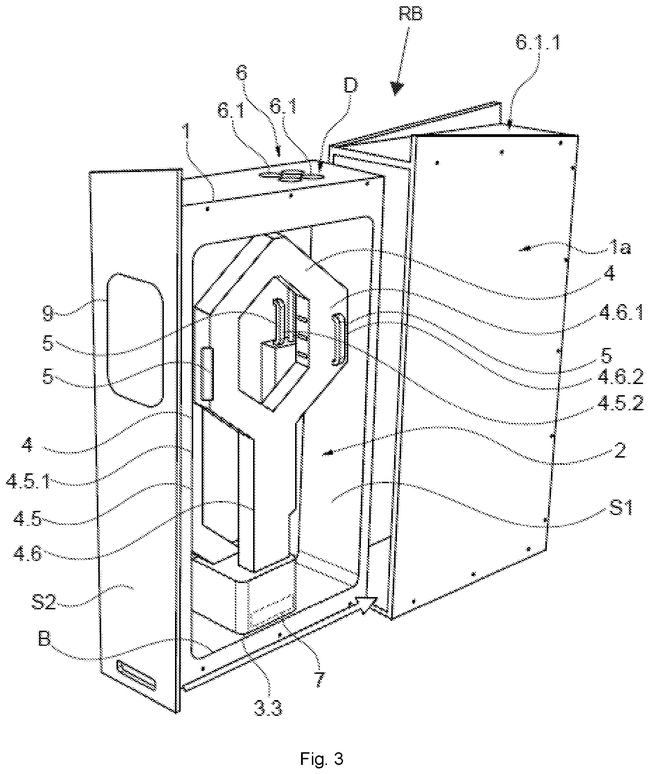

Gemäß Figur 2 und 3 weist das Gehäuse 1 der Box RB eine Kammer 2 (Hauptkammer bzw. Reinigungskammer) mit einer Luftzuführung 3 mit einer in der Front angeordneten Lufteinlass 3.1 und einem alternativ ausgestalteten Grundkörper 4 auf. Der hohl ausgeführte Grundkörper 4 ist an seiner Unterseite mit der Luftzuführung 3 direkt verbunden und erstreckt sich in sich verzweigender Ausführung nach oben und ist in Form eines ersten Adapters 4.5 und eines zweiten Adapters 4.6 ausgebildet, die sich nach oben in die Kammer 2 erstrecken. Der erste und zweite Adapter 4.5, 4.6 des Grundkörpers 4 sind selbstverständlich ebenfalls hohl ausgeführt und weisen eine Luftführung 4.5.1, 4.6.1 auf, mittels derer der, durch das kammerseitige Ende 3.3 geführte Luftstrom zu jedem Adapter 4.5, 4.6 aufteilbar ist und somit in einem ersten Luftstrom in den ersten Adapter 4.5 und in einem zweiten Luftstrom in den zweiten Adapter 4.6 führt.

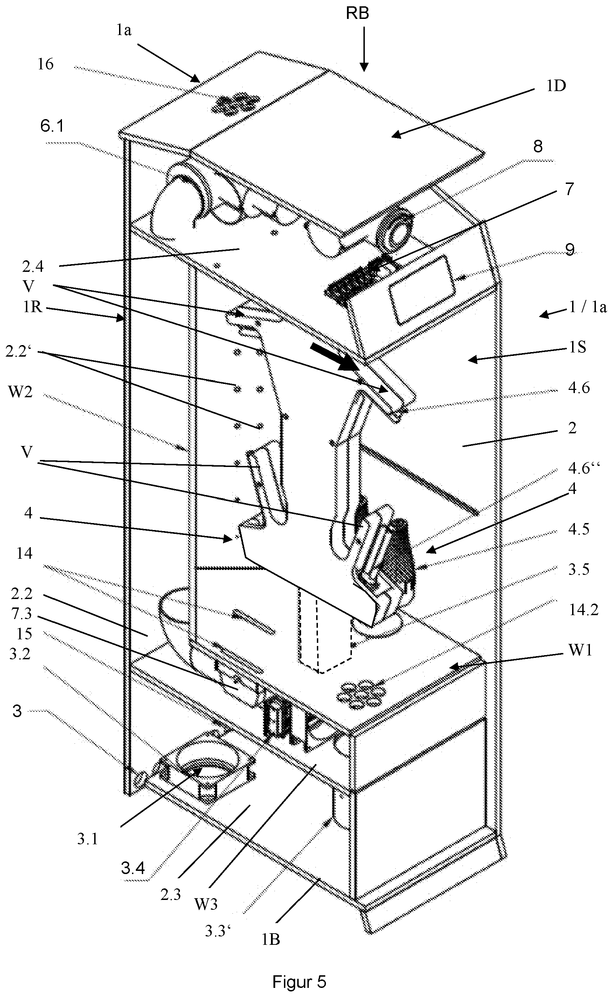

Der erste Adapter 4.5 und der zweite Adapter 4.6 weisen jeweils zwei Verzweigungen auf. Im Bereich jeder Verzweigung ist mindestens eine Austrittsöffnung 4.5.2, 4.6.2 vorgesehen, gemäß Figur 2 insgesamt vier Austrittsöffnungen (zwei je Adapter 4.5, 4.6), in deren Bereich je eine UV-Strahlungsquelle 5 angeordnet ist. Die Austrittsöffnungen können radial oder axial in Bezug auf den einzelnen Luftstrom angeordnet sein. Im vorliegenden Beispiel gemäß Figur 5 ist der erste Adapter Y-förmig (Y) ausgebildet, und weist an seinem kammerseitigen Ende eine erste und zweite UV-Strahlungsquelle 5 auf. Ein derartiger Adapter eignet sich insbesondere für die Positionierung von, einen Hohlraum bildenden Gegenstand in Form von Schuhen, Handschuhen, Atemschutzmasken, Helmen und dergleichen, da diese auf eine einzelne UV-Strahlungsquelle 5 oder zwei UV-Strahlungsquellen 5 überspannend aufgelegt oder aufgesteckt werden.

Der zweite Adapter 4.6 unterscheidet sich in seiner Form vom ersten Adapter 4.5 derart, dass die zwei Luftströme bzw. Luftkanäle wieder zusammengeführt werden, beziehungsweise miteinander verbunden sind. Die Form des zweiten Adapters 4.6 entspricht einem "Koppa" ( 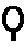 griechisches Alphabet). Die UV-Strahlungsquellen 5 sind im Bereich der zwei aufgeteilten Luftströme angeordnet. Mit einer derartigen Ausgestaltung eines Adapters 4.6 können textile Gegenstände ohne einen weiteren Bügel aufgehangen werden.

In der Kammer 2 (Hauptkammer bzw. Reinigungskammer) ist hier somit ein Grundkörper mitbevorzugt zwei Adaptern angeordnet, wobei zwei Adapter des ersten Adaptertyps 4.5, zwei Adapter des zweiten Adaptertyps 4.6 oder je ein Adapter des ersten Typs 4.5 und des zweiten Typs 4.6 Anwendung finden. Vorzugsweise sind in dem ersten und zweiten Adapter 4.5, 4.6 die Verkabelungen für den Anschluss der UV-Strahlungsquellen 5 vorhanden.

Die Adapter 4.5, 4.6 können durchgehend symmetrisch und/oder asymmetrisch verteilte Löcher und/oder Durchgänge zur Verteilung der Luft oder des Wasserdampfes aufweisen.

Über dem kammerseitig im Boden B angeordneten Bereich der Luftzuführung 3.3 ist ein sich vertikal erstreckender Bereich eines Luftkanals 4A vorgesehen. An dessen oberes Ende schließen sich der erste und der zweite Adapter 4.5 und 4.6 des Grundkörpers 4 an, die kanalartig ausgebildet sind und durch welche der Luftstrom weiter aufgeteilt wird.

Es ist möglich, dass die Adapter 4.5 und 4.6 mit dem Luftkanal 4A einteilig ausgebildet und lösbar und austauschbar am Boden befestigt sind oder dass die Adapter 4.5 und 4.6 separat ausgebildet und lösbar am oberen Ende des Luftkanals 4A befestigt sind.

In jedem Fall können dann beispielsweide auch nur ein oder zwei oder auch mehr erste Adapter 4.5 oder ein oder zwei oder auch mehr zweite Adapter oder eine unterschiedliche Anzahl und Kombination der Adapter verwendet werden, je nachdem, welcher Gegenstand gereinigt werden soll.

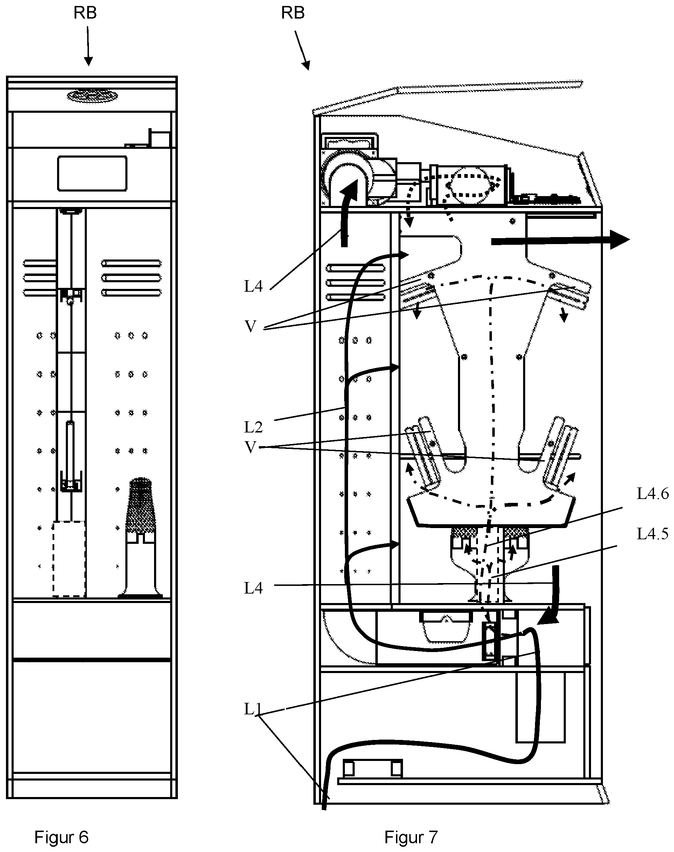

Gemäß Figur 5 und 6 ist der Wassertank 7 der Box RB mit einem in diesem angeordneten nicht dargestellten Heizelement im Bereich des Übergangs zwischen dem kammerseitigen Ende 3.3 der Luftzuführung und dem ersten und zweiten Adapter 4.5, 4.6 angeordnet, hier in dem Bereich des Luftkanals 4A. Das Wasser wird derart erwärmt, dass Wasserdampf entsteht, wobei der Wasserdampf über eine nicht bezeichnete Leitung der Luftzuführung 3 zugeführt wird.

Es erfolgt eine Vermischung von erwärmter Luft und dem erzeugten Wasserdampf, wobei das Gemisch über das kammerseitige Ende 3.3 den in den Adaptern ausgebildeten Hohlräumen zugeführt wird und über diese zu den UVC-Strahlungsquellen geleitet wird.

Durch die mit Feuchte angereicherte Luft erfolgt eine Glättung des zu reinigenden Gegenstandes, wodurch ein späteres Bügeln entfällt.

Auch der zusätzliche Einsatz einer speziellen Bügelflüssigkeit ist möglich, sodass anstelle von Wasser die Bügelflüssigkeit in den schematisch angedeuteten Wassertank 7 gefüllt wird.

Das Glätten der Gegenstände mittels Wasserdampf erfolgt dabei bevorzugt nach der Abschaltung der UV-Strahlungsquellen und somit nach der Behandlung des Gegenstandes mit der UV-Strahlung.

Für eine effektivere Reinigung wird die Luft im Bereich der Luftzuführung 3 wie bereits zuvor beschrieben erwärmt oder abgekühlt oder auch abwechselnd erwärmt und gekühlt. Dafür ist im Bereich der Luftzuführung 3 die Heizeinrichtung 3.4 angeordnet. Alternativ kann auch ein Heizlüfter als Kombination aus Heizeinrichtung 3.4 und Lüfter 3.2 eingesetzt werden. Die durch die Luftzuführung 3 strömende Luft wird im Bereich der Heizeinrichtung 3.4 erwärmt oder gekühlt.

Die Temperatur ist in einem Temperaturbereich von -50 °C bis 100 °C, bevorzugt in einem Temperaturbereich von -30°C bis 60°C, besonders bevorzugt in einem Temperaturbereich zwischen - 10 °C und 50 °C einstellbar und/oder veränderbar.

In einer weiteren Ausgestaltung ist im Bereich der Luftzuführung 3 eine Duftkapsel 8 zur Erzeugung eines Frischeduftes der Gegenstände in die Luftzuführung 3 angeordnet. Die freigesetzten Duftstoffe können mit der von der Luftzuführung 3 angesaugten Luft zugesetzt und in die Kammer 2 (Hauptkammer bzw. Reinigungskammer) geleitet werden.

Gemäß den vorgegangenen Figuren weist die Kammer 2 (Hauptkammer bzw. Reinigungskammer) gemäß dem Ausführungsbeispiel in den Figuren 5 und 6 eine Absaugeinrichtung 6 im Deckenbereich der Kammer 2 auf. Die Absaugeinrichtung 6 ist bevorzugt im oberen Bereich der Vorrichtung angeordnet und weist einen nach oben gerichteten und nach außen führenden Luftauslass 6.1 auf, in dem ebenfalls ein nicht dargestellter Lüfter. Die Absaugeinrichtung 6 kann ebenfalls mit einem nicht dargestellten austauschbaren Filter ergänzt werden, sodass die verschmutzte Luft vor Austritt aus der Kammer gereinigt und das Ozon entfernt werden kann. Der Filter ist bevorzugt im Bereich des Lüfters und der Absaugeinrichtung 6 angeordnet.

Auch gemäß der Figur 2 und 3 kann mit der Absaugeinrichtung 6 und der Luftzuführung 3 ein wechselnder Druck in der Kammer 2 erreicht werden, wobei die Absaugeinrichtung 6 derart ausgebildet sein kann, dass ebenfalls ein Vakuum in der Kammer 2 erzeugbar ist. Für eine derartige Ausgestaltung ist die Kammer 2 vorzugsweise luftdicht ausgebildet.

Aus Figur 3 ist ersichtlich, dass die Absaugeinrichtung 6 mit einen oder mehreren Luftauslässen 6.1 auch im Deckel D angeordnet sein kann. Dann befinden sich in der nicht bezeichneten Oberseite des Schrankkorpusses 1a Austrittsöffnungen 6.1.1, über welche die abgesaugte Luft austreten kann. Im eingeschobenen Zustand des Gehäuses 1 befinden sich die Austrittsöffnungen 6.1.1 des Schrankkorpusses 1a über den Luftauslässen 6.1 im Deckel D des Gehäuses 1.

Des Weiteren ist im Bereich des Bodens B der Kammer 2 ein die Anordnung eines zusätzlichen Drucksystems 11 möglich, mittels dessen die Luftzuführung 3 und die Absaugeinrichtung 6 zur Erzeugung eines Unter- oder Überdruckes unterstützt wird.

Der Druck in der Kammer 2 kann in einem Druckbereich zwischen 0,001 bar bis 10 bar, insbesondere zwischen 0,1 bar und 2 bar einstellbar und/oder veränderbar sein.

Der Einsatz eines Filters ist besonders empfehlenswert da austretendes Ozon bereits in geringen Konzentrationen gesundheitsschädlich durch Reizungen der Atemwege ist.

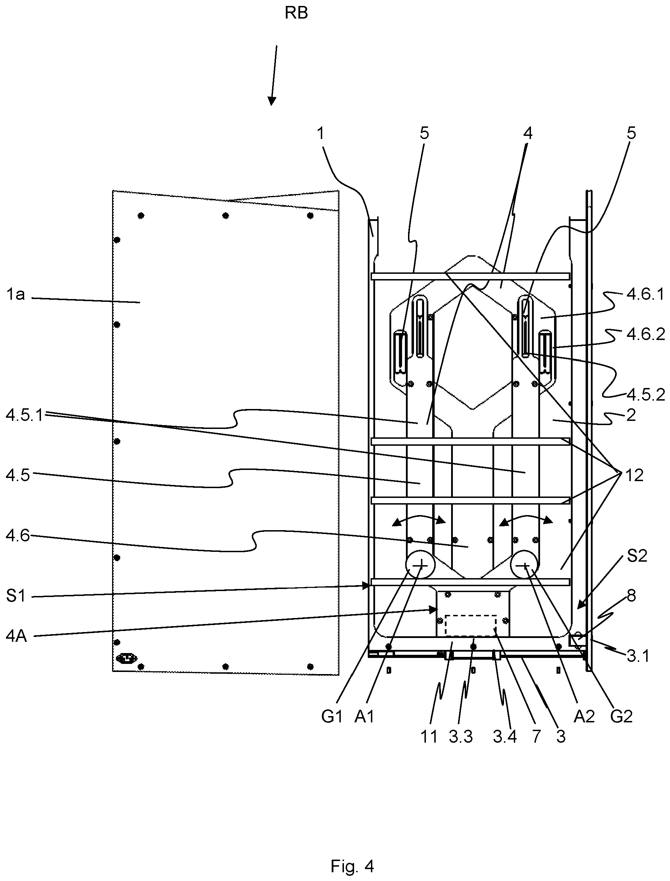

Figur 4 zeigt eine Darstellung einer Box RB, ähnlich wie Figur 6 jedoch ohne Deckel in dem Einschub und dafür mit Querstreben 12 zwischen den Seiten S1 und S2, welche die Seiten S1, S2 zueinander stabilisieren und dafür sorgen, dass die zu reinigenden Gegenstände nicht seitlich aus dem Gehäuse 1 herausstehen und das Einschieben in den Korpus verhindern. Es ist hier möglich, die zu reinigenden Gegenstände von oben einzulegen.

Die hier nicht dargestellte Absaugung ist dann wie in Figur 1 in dem vorderen Seitenteil S2 oder auch in dem rückwertigen Seitenteil S2 angeordnet, wobei dann in dem Schrankkorpus 1a in der Rückwand entsprechende Luftauslässe vorhanden sind.

Bei dieser Variante ist angedeutet, dass sich an den Luftkanal 4A des Grundkörpers 4 vorgesehen der erste und der zweite Adapter 4.5 und 4.6 lösbar anschließen.

Anstelle von Querstreben können sich auch ein oder mehrere plattenartige Elemente zwischen den Seitenteilen S1, S2 erstrecken.

Derartige Querstreben oder plattenartige Elemente können auch bei den vorgenannt beschriebenen Varianten eingesetzt werden.

Weiterhin ist es gemäß Figur 7 möglich, dass beispielsweise der erste Adapter 4.5 in den beiden sich nach oben erstreckenden Bereichen der Luftführungen 4.5.1 jeweils ein Gelenk G1 und G2 aufweist. Der sich hier dargestellte sich beispielsweise in einer Ausgangsposition vertikal nach oben erstreckende Bereich ist dadurch um eine horizontale Achse A1, A2 schwenkbar ausgebildet, was durch die Doppelpfeile angedeutet ist. Dadurch können die oberen Enden der beiden Luftführungen 4.5.1 des Adapters 5.5 aufeinander zu und voneinander weg geschwenkt werden und somit deren Abstand den aufzunehmenden Gegenständen angepasst werden.

Mit der erfindungsgemäßen Vorrichtung wird der der Kammer 2 (Hauptkammer bzw. Reinigungskammer) zugeführte Luftstrom über die Kanäle, die durch die Adapterelemente gebildet werden in der Kammer aufgeteilt und jeweils zu einer UV-Strahlungsquelle 5 geführt.

Die Anzahl der in einem Adapter ausgebildeten Kanäle entspricht dabei der Anzahl der am Adapter vorgesehenen UV-Strahlungsquelle 5
Es ist somit auch möglich, einen Grundkörper 4 bzw. Adapter nicht nur mit zwei, sondern auch mit drei, vier oder mehr sich nach oben erstreckenden Verzweigungen vorzusehen, die jeweils den Luftstrom an eine UV-Strahlungsquelle 5 führen.

Bevorzugt befinden sich die UV-Strahlungsquelle 5 an den Luftaustrittsbereichen der Adapter.

Dabei bilden die Adapter auch die Aufnahmen für die zu reinigenden Gegenstände.

Die Adapter können dabei so ausgeführt sein, dass der Abstand der oberen Bereiche, die die zu reinigenden Gegenstände aufnehmen, veränderbar ist.

Es ist möglich, mehrere der erfindungsgemäßen im Wesentlichen quaderförmigen Vorrichtungen nebeneinander und/oder übereinander batterieartig anzuordnen.

Dabei können die Vorrichtungen auch untereinander verbunden werden.

Es ist weiterhin auch alternativ möglich, einen Schrankkorpus mit einer schwenkbaren oder seitlich verschiebbaren Tür vorzusehen, wobei dann in dem Schrankkorpus die Kammer / Reinigungskammer ausgebildet ist.

In Abhängigkeit des zu reinigenden Gegenstandes können die einzelnen Parameter wie Druck, Temperatur, UV-Strahlung und Ozon eingestellt werden. Dabei empfiehlt sich beispielsweise bei der Reinigung von Leder eine Nutzung der maximalen Leistung der Vorrichtung, während beispielsweise Kaschmir nur mit einer schonenden Behandlung erfolgen sollte.

In den Figuren 5 bis 7 wird eine weitere Variante einer erfindungsgemäßen Vorrichtung dargestellt. Hier ist kein in einen Schrankkorpus 1a der Box RB einschiebbares Gehäuse 1 vorgesehen, sondern das Gehäuse1 bildet den Schrankkorpus 1a. Die Kammer 2 (Hauptkammer bzw. Reinigungskammer) ist hier somit nicht herausziehbar, sondern in dem Schrankkorpus 1a ausgebildet. Die Vorrichtung weist somit einen Korpus 1a auf, in welchem die Kammer 2 (Reinigungskammer) ausgebildet ist. Hier sind in der Kammer 2 zwei Grundkörper 4 zur Aufnahme von Gegenständen vorgesehen und zwar ein erster Grundkörperr 4.5 zur Aufnahme von einem Paar Schuhe oder von einem Paar Handschuhen oder Schutzmasken und ein zweiter Grundkörper 4.6 zur Aufnahme von Kleidung in Form von Hemden, Kitteln, Jacken und dergleichen. Der erste und zweite Grundkörper 4.5, 4.6 sind hohl ausgeführt und weisen in ihrem Inneren eine durch die Hohlstruktur führende nicht ersichtliche Luftzuführung auf. Der über eine Leitung einströmende Luftstrom ist je Adapter 4.5, 4.6 in einen ersten und zweiten Luftstrom aufteilbar und strömt an den hier nicht ersichtlichen und nicht bezeichneten UV-Lampen der Adapter 4.5, 4.6 vorbei. Diese UV-Lampen sind an Austrittsbereichen der Adapter 4.5, 4.6 angeordnet, so dass der aus den Adaptern 4.5, 4.6 austretende Luftstrom an den UV-Lampen vorbeiströmt.

Der erste Grundkörper 4.5 verzweigt sich nach oben in zwei nicht bezeichnete Adapterelemente mit Ausströmöffnungen (nicht dargestellt) , in welchen UV-C Lampen angeordnet sind und besitzt eine Größe, die es gestattet, dass ein Paar Schuhe, Handschuhe, Schutzmasken und dergleichen darauf zu positionieren, so dass sich die UV-Lampen zumindest teilweise in den zu reinigenden Gegenständen befinden und der aus dem ersten Adapter 4.5 austretende Luftstrom mit dem durch die UV-Lampe gebildeten Ozon in den Gegenstand eindringt und diesen ggf. auch durchdringt (bei textilem Material).

Der zweite Grundkörper 4.6 ist ebenfalls hohl ausgeführt und weist an seinem oberen Bereich bügelförmig ausgebildet, so dass darüber ein Hemd, eine Jacke, ein Kittel, Gegenstände der Tab. 2 und dergleichen aufgehangen werden kann. Dazu erstrecken sich am oberen Bereich des zweiten Grundkörpers 4.4 zwei seitlich voneinander wegweisende und nach unten abgewinkelte Vorsprünge V mit nicht bezeichneten Öffnungen, in welchen die UVC-Lampen angeordnet sind.

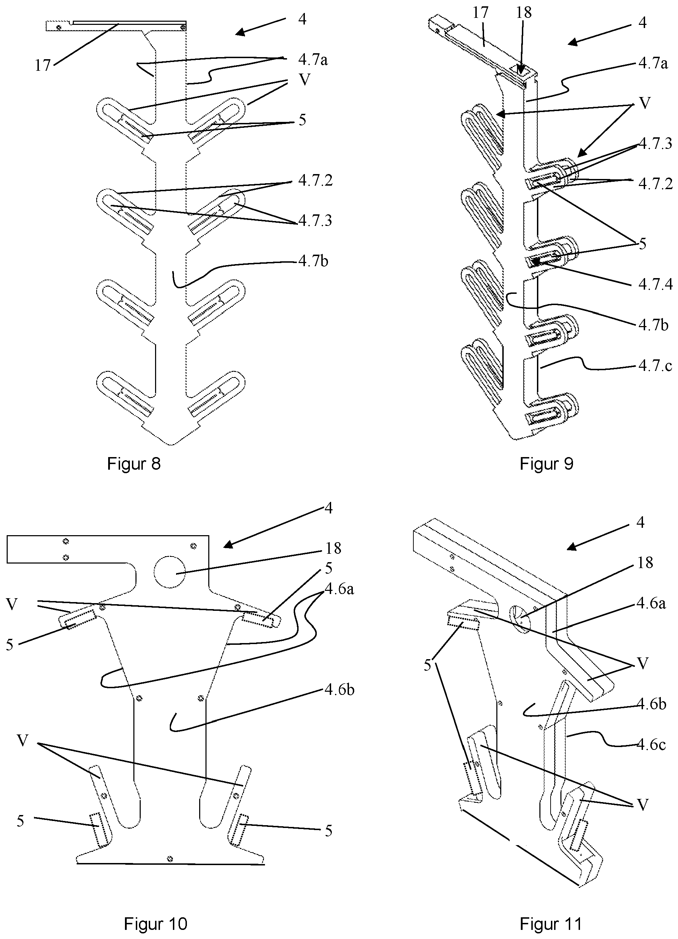

Bevorzugt der zweite Grundkörper 4.6 ist z.B. über eine obere schienenförmige Aufhängung 13 (siehe Figur 10) aus der Vorrichtung in Richtung des fetten Pfeiles herausziehbar, so dass die Kleidung oder andere Gegenstände besser darüber positioniert werden können. An den sich gegenüberliegenden Vorsprüngen V des bügelförmigen Bereiches befinden sich nicht bezeichnete Austrittsöffnungen und innerhalb jedes Endes eine UV-Lampe. Nach dem Aufnehmen der Gegenstände wird der zweite Adapter 4.6 wieder in die Vorrichtung zurückgeschoben. Bei Reinigungsvorgang wird der Luftstrom entweder von oben oder von unten in den zweiten hohl ausgeführten Adapter 4.6 geleitet, strömt an nicht dargestellten UV-Lampen vorbei und aus den Austrittsöffnungen im bügelförmigen Bereich heraus. An dem unteren Bereich des zweiten Grundkörpers 4.6 sind zusätzlich zwei nach oben weisende Vorsprünge V vorgesehen. Diese weisen seitliche Schlitze 4.6" auf, durch welche mit Ozon angereicherte Luft in die Kammer 2 strömen kann. Bei längeren Kleidungsstücken ist dadurch auch eine Reinigung des unteren Bereiches gewährleistet, wenn diese bis über die unteren Vorsprünge V reichen.

Unter der Kammer 2 und seitlich davon erstreckt sich in dem Korpus 1a eine zweite Kammer 2.2. Diese ist durch eine horizontale Wand W1 und eine vertikale Wand W2 von der zweiten Kammer 2.2 getrennt. Unter der zweiten Kammer 2.2 ist eine dritte Kammer 2.3 angeordnet, die von der zweiten Kammer 2.2 durch eine horizontale Wand W3 getrennt ist. Über der ersten Kammer 2 und dem senkrechten Bereich der zweiten Kammer 2.2 befindet sich eine von diesen durch eine horizontale Wand W4 getrennte vierte Kammer 2.4. Der Schrankkorpus weist unter der dritten Kammer einen Boden 1B, eine Rückwand 1R, einen Deckel 1d, zwei Seitenwände 1S (von denen nur eine sichtbar ist) und eine unterhalb des Displays 9 angeordnete Tür (hier nicht dargestellt) auf.

Um die Innengestaltung der Vorrichtung darstellen zu können wurden eine Seitenwand und die Tür nicht eingezeichnet.

Seitlich im unteren Bereich der dritten Kammer 2.3 befinden sich Aussparungen 3 für die Luftzuführung. In der unteren dritten Kammer 2.3 ist weiterhin eine Luftzuführung 3.1 in dem Boden 1B und eine Einlasspumpe bzw. eine Lüfter 3.2 vorgesehen. Zur zweiten Kammer 2.2 führt von der dritten Kammer 2.3 eine Einlassregulierung 3.3' und über diese die hier nicht bezeichnete Luftzuführung in die zweite Kammer 2.2. Die Luft wird in der zweiten Kammer 2.2 mittels eines Heizelementes 3.4 auf die gewünschte / voreingestellte Temperatur erwärmt. Die Luft strömt in dem seitlichen Bereich der zweiten Kammer 2.2 nach oben in die vierte Kammer 2.4 und in der vertikalen Trennung W3 zwischen der Kammer 2 und der zweiten Kammer 2.2 befinden sich Durchbrüche 2.2', durch welche ein Teil der angesaugten Luft in die Kammer 2 eintreten kann.

Die angesaugte Luft wird in das Innere der Grundkörper 4.5, 4.6 geführt. Dies erfolgt entweder von unten aus der zweiten Kammer 2.2 über eine Zuleitung 3.5 (hier gestrichelt dargestellt) oder von oben aus der vierten Kammer 2.4 über eine hier nicht dargestellte Zuleitung.

In dem Boden, bzw. der horizontalen Wand W3 der zweiten Kammer befinden sich Öffnungen 14, durch welche Wasserdampf zur Glättung der Gegenstände zugeführt werden kann. Dieser wird bevorzugt mit Druck aus einem dafür vorgesehenen Verdampfer 7.3 zugeführt. Die Wasserzuführung zum Verdampfer 7.3 erfolgt aus einem Wassertank 7, der hier in der oberen vierten Kammer 2.4 angeordnet und über eine nicht dargestellte Leitung mit dem Verdampfer 7.3 verbunden ist. Der heiße Wasserdampf dient zusätzlich zur Abtötung von Viren, Bakterien, Pilzen und anderen Keimen. Weiterhin können in dem Boden (Wand W2) der Kammer 2 Öffnungen 14.2 zur Erzeugung einer Umluft zwischen der Kammer 2 und der zweiten Kammer 2.2 vorgesehen sein.

In der dritten Kammer 2.3 ist weiterhin ein Anschluss 15 für eine Vakuumpumpe vorgesehen, durch welche in der Kammer 2 ein Vakuum erzeugbar ist. Das Vakuum wird dabei in der Kammer 2 und in der zweiten Kammer 2.2 ausgebildet.

Wie bei dem vorgenannt beschriebenen Beispiel ist auch hier eine Duftkapsel 8 vorgesehen, die hier in der vierten Kammer 2.4 angeordnet ist (optional).

Die Elektronik befindet sich ebenfalls in der vierten Kammer 2.4 und an deren Vorderseite die Steuerung 9 mit einem entsprechenden Bediendisplay vorgesehen. Der Deckel 1D der vierten Kammer 2.4 ist bevorzugt nach oben schwenkbar. In dem Deckel 1D sind Öffnungen 16 angeordnet, die zur Kühlung der Elektronik dienen und in/unter denen ein oder mehrere Lüfter angeordnet sein können.

Die Kammern zumindest die Kammer 2, wird luftdicht verschlossen, bevorzugt über eine Tür mit einem entsprechenden Schließmechanismus.

Die Temperatur in der zweiten Kammer liegt ebenfalls in einem Temperaturbereich von -50 °C bis 100 °C, bevorzugt in einem Temperaturbereich von -30°C bis 60°C, besonders bevorzugt in einem Temperaturbereich zwischen -10 °C und 50 °C.

Der Druck in der Kammer 2 kann auch hier in einem Druckbereich zwischen 0,001 bar bis 10 bar, insbesondere zwischen 0,1 bar und 5 bar einstellbar und/oder veränderbar sein.

Es können auch bei dieser erfindungsgemäßen Ausführungsvariante mehrere erste und zweite Adapter vorgesehen sein.

Besonders vorteilhaft ist hier, dass die Grundkörper separat aus der Vorrichtung herausziehbar sind, wodurch diese sehr einfach mit dem zu reinigenden Gegenstand in Form von Jacken, Kitteln und dergleichen bestückt werden können.

Figur 7 zeigt den Luftstrom (in der Vorrichtung. Die Luft tritt in die dritte Kammer 2.3 ein (Hauptstrom L1 - Durchgangslinie) und gelangt über die Einlassregulierung 3.3 in die zweite Kammer 2.3. Von dort aus wird dieser aufgeteilt in
- einen ersten Luftstrom L4.5 (gestrichelt dargestellt), der von unten in den ersten Grundkörper 4.5 führt,
- einen zweiten Luftstrom L4.6 (Strichpunktlinie), der von unten in den zweiten Grundkörper 4.6 führt,
- einen dritten Luftstrom L2 (dünnere Durchgangslinie), der über die zweite Kammer 2.2 seitlich nach oben geführt wird und seitlich in die erste Kammer 2 über die Durchbrüche und Lüftungsschlitze eintritt,
- einen vierten Luftstrom L4 (dargestellt durch die fetten Pfeile), der eine Umluft zwischen erster Kammer 2 (Hauptkammer/Reinigungskammer), zweiter Kammer und vierter Kammer L.4 erzeugt, bevorzugt wenn in der Kammer 2 und der zweiten Kammer 2.2 kein Vakuum herrscht.

Der zweite Luftstrom L4.5 tritt aus Durchtrittsöffnungen (nicht bezeichnet) des ersten Grundkörpers 4.5 nach dem vorbeiströmen an der UV-Lampe (nicht dargestellt) aus,
Der dritte Luftstrom L4.6 tritt nach dem Hindurchströmen durch den zweiten Grundkörper 4.6 ebenfalls an Austrittsöffnungen in den Vorsprüngen V am oberen Bereich bzw. neben den hier unten befindlichen Vorsprüngen V im Bereich der UV-Lampen aus.

Wie bereits vorgenannt beschrieben können die Grundkörper 4 (4.5, 4.6) unterschiedlich ausgebildet sein und auch mehrere unterschiedliche in einer Vorrichtung integriert sein, vorzugsweise auswechselbar.

Dies wird dann über eine Schnittstelle zwischen Grundkörper 4, 4.5, 4.6 und Schrankkorpus bzw. Vorrichtung realisiert, wobei die Schnittstelle ein einfaches Entnehmen eines Grundkörpers und ein Einsetzen eines neuen Grundkörpers gewährleisten soll.

Die Schnittstelle ist bevorzugt in Form eines Schienensystems ausgebildet, über welches das Aufnahmeelement 4 (4.5, 4.6) auch aus der Vorrichtung bzw. der Box / des Schrankkorpus soweit herausziehbar ist, dass dieser einfach mit den zu reinigenden Gegenständen bestückt werden kann, bzw. um die gereinigten Gegenstände zu Entnehmen.

Alternativ kann der Luftstrom über eine doppelte seitliche Wand der Kammer 2 in die Kammer einfließen und in die Adapter, welche um 90° gedreht sind, eintreten und wie im vorherigen Text geführt werden.

In den Figuren 8 bis 11 sind beispielhaft zwei unterschiedliche Grundkörper 4 dargestellt, welche eine Vielzahl von sich gegenüberliegenden und übereinander angeordneten Aufnahmen in Form von Vorsprüngen V aufweisen.

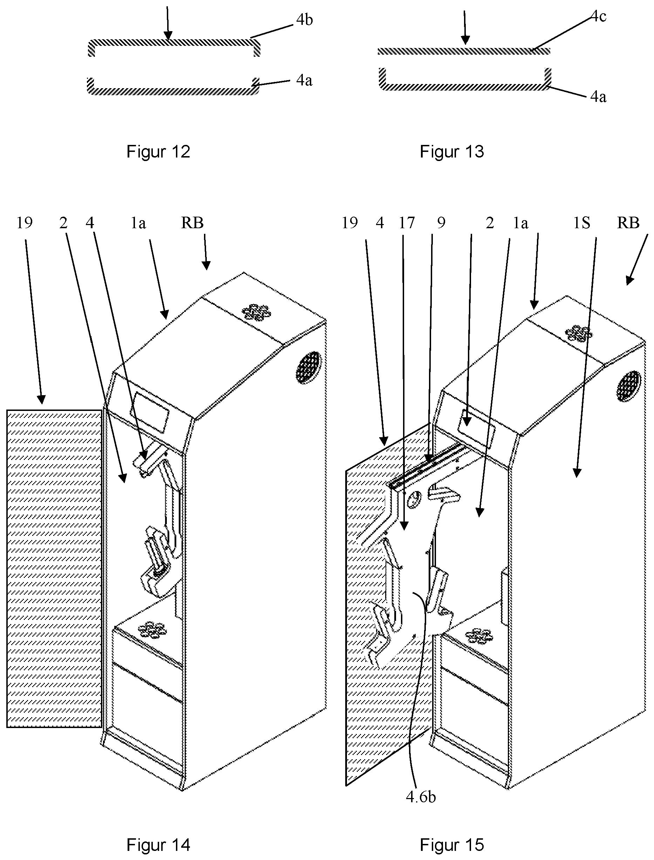

Gemäß Figur 8 und 9 weist ein hohl ausgeführter und sich vertikal erstreckender Grundkörper 4 an sich gegenüberliegenden Seiten 4.7a eine Vielzahl von sich seitlich und schräg nach oben erstreckenden Vorsprüngen V auf. Die Vorsprünge V sind hier jeweils paarweise in Form von zueinander parallelen Stegen 4.7.2 (siehe Figur 12) ausgebildet. In jedem Steg ist ein schlitzartiger Durchbruch 4.7.3 vorhanden.

Zwischen jeweils zwei paarweise angeordneten Stegen 4.7.2 ist in der jeweiligen Seite 4.7.a des Grundkörpers 4 eine Durchströmöffnung 4.7.4 vorhanden. Weiterhin ist zwischen den zwei Stegen 4.7.2 die UV-Strahlungsquelle 5, bevorzugt eine UVC-Lampe angeordnet.

Zwischen den Beiden Seiten 4.7a erstreckt sich jeweils eine Vorderseite 4b und eine Rückseite 4.7.c.

Bevorzugt werden Vorderseite 4.7b und Rückseite 4.7c mit den sich daran anschließenden Stegen aus einer Platte zugeschnitten und sind über die Seiten 4.7.a zueinander beabstandet.

Vorderseite 4.7b, Rückseite 4.7c und die Seiten 4.7.a werden bevorzugt aus Holz, insbesondere Sperrholz, gefertigt und miteinander verbunden - verleimt und/oder verschraubt -, so dass der Hohlkörper in Form des Grundkörpers 4 gebildet wird.

An der Oberseite des dritten Adapters 4.7 befindet sich eine Schiene 17, über welche der dritte Adapter 4.7 mit dem hier nicht dargestellten Schrankkorpus in Verbindung bringbar und in diesen Ein- und Ausschiebbar ist. Weiterhin befindet sich am oberen Ende eine Eintrittsöffnung 18, durch welche während des Betriebs Luft in den dritten Adapter 4.3 strömt.

Beim Reinigungsvorgang strömt somit Luft über die Eintrittsöffnung 18 in den Grundkörper 4 in dessen Hohlraum und über die Durchströmöffnungen 4.7.4 nach außen und dabei an den UV-Strahlungsquellen 5 vorbei und zwischen den Vorsprüngen 4.7.1 und somit den Stegen 4.7.2 und deren Durchbrüche 4.7.3 hindurch. Sind ein oder mehrere Gegenstände über einem oder mehreren Vorsprüngen V positioniert, werden diese Gegenstände durch den Ozon mitführenden Luftstrom und das UVC-Licht ggf. in Verbindung mit einer eingestellten Temperatur und/oder einem Druck, bevorzugt einen Unterdruck, in der hier nicht dargestellten Kammer 2 (Reinigungskammer) zuverlässig desinfiziert.

Die Figuren 10 und 11 zeigen die Eizelteildarstellung einer weiteren Variante eines Grundkörpers 4 für einen oder mehrere Gegenstände gemäß Figur 7, bei dem die Schienen nicht dargestellt sind.

Dieser ist ebenfalls als Hohlkörper ausgebildet und aus Platten, insbesondere aus Holz oder Kunststoff, bevorzugt jedoch aus Sperrholz zusammengesetzt.

Es weist Seiten 4.6.a sowie eine Vorderseite 4.6.b und eine Rückseite 4.6.c auf, die miteinander verbunden sind.

In der Vorderseite 4.6.a und der Rückseite 4.6.b sind im oberen Bereich Durchbrüche als Eintrittsöffnungen 18 angeordnet.

Die Vorderseite 4.6b ist dabei bevorzugt lösbar mit den Seiten 4.6a verbunden. Auch hier weist der Grundkörper 4 Vorsprünge V auf. Die Vorsprünge V schließen sich paarweise an den Grundkörper 4 an. Dabei sind zwei Vorsprünge V einander abgewandt, gegenüberliegend im oberen Bereich und zwei Vorsprünge V einander abgewandt, gegenüberliegend im unteren Bereich des Grundkörpers 4 angeordnet.

Die beiden oberen Vorsprünge V sind einander entgegengesetzt etwas nach unten abgewinkelt, so dass eine bügelförmige Aufnahme für Kleidungsstücke in Form von Jacken, Mäntel, Kittel und dergleichen gebildet wird. Die beiden unteren Vorsprünge V ragen in einem Winkel nach oben und außen. Im Bereich der Vorsprünge V sind die hier gestrichelt dargestellten UV-Strahlungsquellen 5 angeordnet. Bevorzugt befinden diese sich innerhalb oder im Bereich von hier nicht dargestellten Durchström Öffnungen des Grundkörpers vor den Vorsprüngen V. In den Vorsprüngen V sind hier nicht sichtbare Durchbrüche angeordnet, durch welche die Luft ausströmt.

Über den unteren Vorsprüngen können Schuhe, Handschuhe, Atemschutzmasken und dergleichen positioniert werden.

Bei längeren Kleidungsstücken, die über die unteren Vorsprünge V reichen, werden die Kleidungsstücke auch an/in ihrer Unterseite sicher und zuverlässig desinfiziert.

Innerhalb des Grundkörpers 4 oder an diesem außen entlang verlaufen Kabel, über welche die Stromzufuhr zu den UV-Strahlungsquellen 5 (bevorzugt UVC-Lampen, insbesondere UVC-LEDs) realisiert wird. Dies wird auch bei den anderen Bauformen von Grundkörpern so umgesetzt. Ansonsten entspricht der Aufbau des Grundkörpers 4 der in Figur 5 dargestellten Ausführung.

Bevorzugt sind die Schnittstellen von Aufnahmeelementen zum Schrank-Korpus 1a bei den unterschiedlichen Grundkörpern gleich ausgebildet um deren Austauschbarkeit zu gewährleisten.

In Figur 12 ist angedeutet, dass der als Hohlkörper ausgebildete Grundkörper 4 aus zwei Halbschalen 4a, 4b gebildet werden kann.

In Figure 13 ist als Prinzipskizze dargestellt, dass der Grundkörper 4 aus einer Halbschale 4a und einer Platte 4c zusammengesetzt ist.

Beide Teile (Halbschalen) 4a, 4b, bzw. 4a, 4c werden miteinander verbunden z.B. unlösbar durch Kleben, oder Schweißen oder auch mittels einer lösbaren Verbindung, bei Kunststoff beispielsweise durch ein Clipsystem oder bei Blech z.B. durch Verschraubungen.

Der Grundkörper 4 besteht in diesem Fall beispielsweise aus Kunststoff oder Blech und die Halbschale wird beispielsweise durch Tiefziehen hergestellt.

Bei Verwendung von Kunststoff könnte das Aufnahmeelement auch einteilig durch Blasformen erzeugt werden.

Figur 14 zeigt die Vorrichtung in Form einer Box RB deren Schrankkorpus 1a an der Vorderseite mittels einer Tür 19 verschließbar ist, in geöffneter Position der Tür 19. Man erkennt in der Box RB einen in der Kammer 2 angeordneten Grundkörper 4.

Gemäß Figur 15 wurde der Grundkörper 4 mittels Schienen 17 aus der Kammer 2 herausgezogen und kann nun bestückt werden. Die Vorderseite 4.6b und die hier nicht bezeichnete Rückseite des Grundkörpers 4 sind dabei im Wesentlichen parallel zu den Seitenwänden 1S des Schrankkorpus 1a der Box RB ausgerichtet. Nach dem zurückschieben des zweiten Grundkörpers 4 in die Kammer 2 wird die Tür 19 geschlossen (vorzugsweise luftdicht) und über das Display 9 der Steuerung können die für die Reinigung erforderlichen Parameter eingestellt und der Reinigungsvorgang gestartet werden. Alternativ ist es möglich, mittels einer App über ein Mobilgerät (z-B. Laptop oder Smartphone) den Reinigungsprozess einzustellen, zu starten und zu beenden und auch das Reinigungsgerät abschließen, aufschließen und über den Status und die Beendigung des Reinigungsprozesses zu informieren.

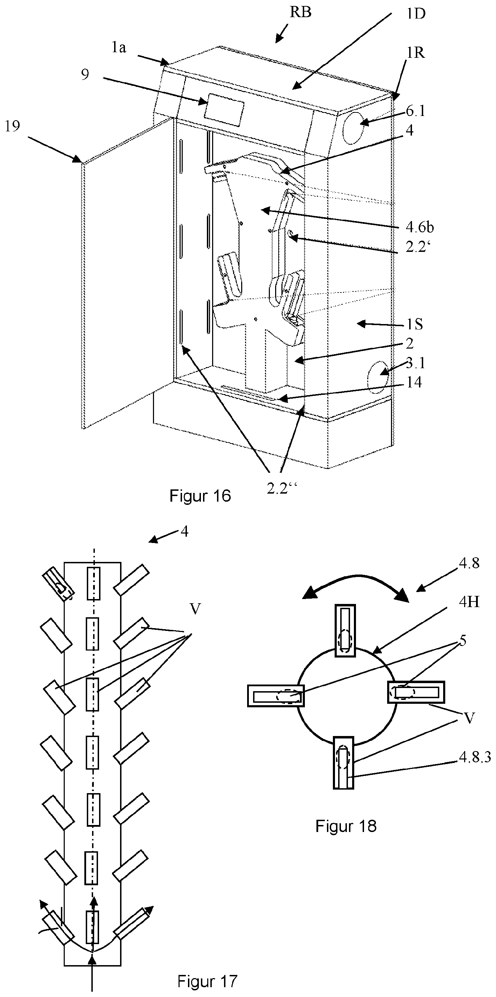

Figur 16 zeigt eine weitere Variante einer Box RB, bei welcher ein Grundkörper im Vergleich zu den Figuren 14 und 15 mit seiner Vorderseite 4.6b im Wesentlichen parallel zur Rückwand 1R des Schrankkorpus 1a ausgerichtet ist. Auch hier kann der Grundkörper 4 über eine Schnittstelle austauschbar und/oder aus der Vorrichtung herausziehbar sein, was jedoch nicht dargestellt ist. Die Box RB weist hier ebenfalls einen bevorzugt aufklappbaren Deckel 1D auf, der den Zugang zu wechselbaren Funktionselementen in der oberen hier nicht bezeichneten vierten Kammer ermöglicht. Über das Display 9 erfolgt die Eingabe. Die Front wird durch eine klappare Tür 19 gebildet. Diese kann hier und auch bei den vorgenannten Ausführungsbeispielen aus UV-stabilen Material, bevorzugt Kunststoff, Polymermischungen, beschichtetes Glas oder aus Metall oder Holz bestehen. Der Grundkörper 3 ist ebenfalls als Hohlkörper ausgeführt und bevorzugt zweilagig (aus zwei Halbschalen) ausgebildet. Der Adapter 4.6 weist im Bereich der UV-Strahlungsquellen Luftauslässe in den Gegenstand aus textilem Material auf, was hier jedoch nicht dargestellt ist.

Es ist eine doppelwandige Rückwand 1R mit angepassten Luftdurchbrüchen 2.2' zur Kammer 2 vorgesehen. In den doppelwandig ausgeführten Seitenwänden 1S befindet sich ebenfalls Luftdurchbrüche 2.2' ' zur Kammer 2. Weiterhin befindet sich im Boden der Kammer 2 ein Zusatzauslass für beispielsweise Wasserdampf 14.

In einer Seitenwand 1s befindet sich unten der Lufteinlass 3.1 und oben der Luftauslass 6.1.

Diese konstruktive Ausführung der Vorrichtung, insbesondere des Schrankkorpus kann selbstverständlich auch für eine andere Gestaltung, Anordnung bzw. Aufnahme des Grundkörpers vorgesehen werden.

Insbesondere, wenn in der Kammer 2 ein Unterdruck erzeugt wird, ist es vorteilhaft, wenn die Luft über die doppelte Rückwand 1R die doppelten Seitenwände 1S über die Luftdurchbrüche abgesaugt werden kann.

Auch Einbringen von heißer Luft in die Kammer 2 ist über die Luftdurchbrüche 2.2" möglich.

Das Erzeugen von Unterdruck in der Kammer 2 und das Einbringen von heißer Luft in die Kammer 2 erfolgt dabei nicht zeitgleich.

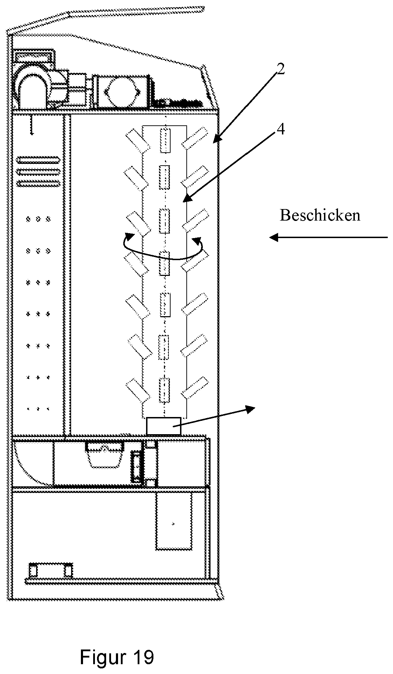

Eine weitere Gestaltung des Grundkörpers 4 ist in Figur 17 in der Vorderansicht, in Figur 18 in der Draufsicht (vergrößert dargestellt) und in Figur 19 bei Anordnung in der Kammer 2 einer Box RB vorgesehen.

Der Grundkörper 4 ist in Form eines rohrförmigen Hohlkörpers ausgebildet, an dessen Umfang sich in einer Ebene jeweils mehrere Vorsprünge 4V erstrecken und mehrere Vorsprünge 4V übereinander angeordnet sind. Die Vorsprünge V weisen schräg nach außen und oben. Jeder Vorsprung 4V schließt sich an einen hier nicht dargestellten Durchbruch des Grundkörpers 4 an. Innerhalb jedes Vorsprungs V ist eine UV-Strahlungsquelle 5, insbesondere eine UVC-Lampe (bevorzugt eine UVC-LED) angeordnet, die hier gestrichelt angedeutet ist. Die Vorsprünge V weisen Durchbrüche 4.8.3 auf und sind an ihrem freien Ende offen ausgeführt.

Die Luftzufuhr ist unten oder oben an dem Adapter 4.8 vorgesehen. Die Luft strömt in den Grundkörper 4 und durch dessen hier nicht sichtbare und nicht bezeichnete Durchströmöffnungen in die Vorsprünge V, an den UVC-Strahlungsquellen 5 vorbei und durch die nach vorn offenen Vorsprünge V und deren Durchbrüche 4.8.3.

Gemäß Figur 19 ist der in der Kammer 2 angeordnete Grundkörper 4 zum Beschicken (hier aus der Pfeilrichtung) an seinem unteren Ende drehbar gelagert.

Es ist auch möglich, dass der Grundkörper 4 auch während der Reinigung in der Kammer 2 rotiert. In diesem Fall ist ein Drehantrieb mit dem Grundkörper 4 wirkverbunden und es ist auch während der Rotation die Stromzufuhr zu den UV-Strahlungsquellen zu gewährleisten, (z.B. über Schleifkontakte oder über Kabel und ein hin- und herdrehen mit einem Drehwinkel kleiner 360°).

Durch das Rotieren des Grundkörpers 4 während der Reinigung können ein schnelleres Trocknen, eine bessere Entfernung von Schmutz und eine bessere Entfernung von allergieverursachenden Pollen realisiert werden,

Insbesondere bei der Reinigung von Schuhen wird durch die während der Rotation auftretenden Zentrifugalkraft die Erhaltung der Schuhform gefördert.

Auch hier kann alternativ oder zusätzlich zur drehbaren Lagerung in der Kammer das Herausziehen über ein Schienensystem vorgesehen werden, welches ggf. auch unten am Aufnahmeelement angeordnet sein kann.

Die Vorsprünge V können bei den vorgestellten Ausführungsvarianten mit dem Grundkörper des Aufnahmeelements einteilig ausgebildet sein oder mit dem Aufnahmeelement Lösbar oder unlösbar verbunden sein. Eine lösbare Verbindung ermögliche ein Austauschen der Vorsprünge.

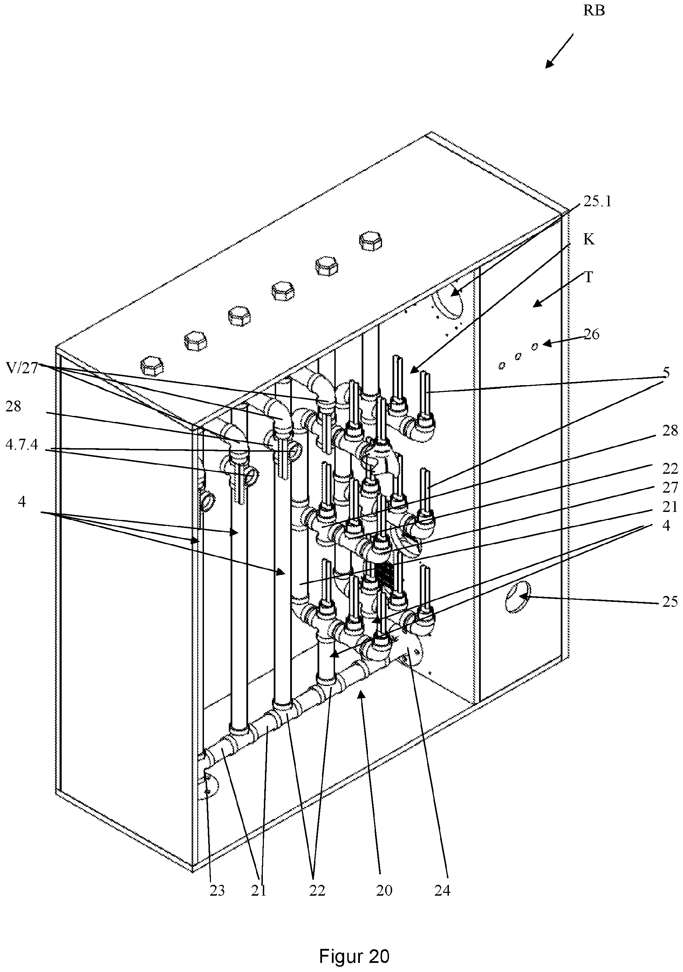

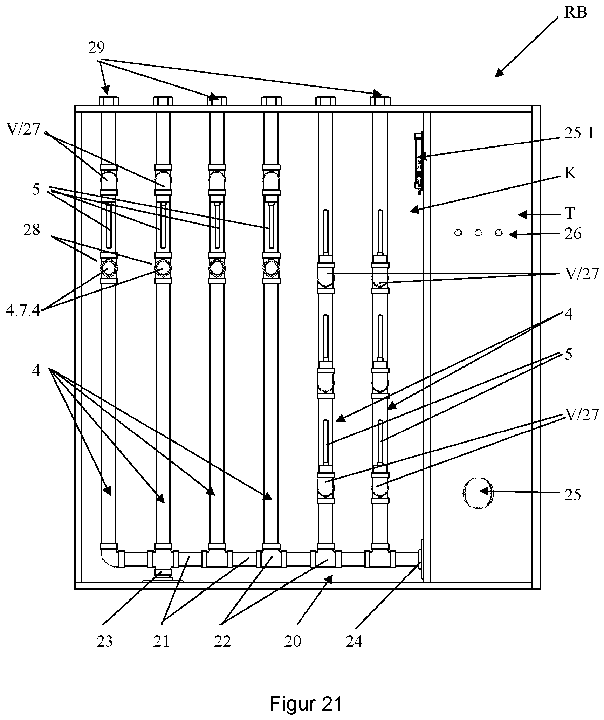

Die Figuren 20 und 21 zeigen eine Vorrichtung in Form einer Box RB mit einer Reinigungskammer K in welcher mehrere rohrförmige Grundkörper 4 angeordnet sind, die nebeneinander angeordnet sind und sich vertikal nach oben erstrecken und hier beispielhaft an ihren unteren Enden miteinander durch eine Verbindungsleitung 20 verbunden sind.

Neben der Reinigungskammer K befindet sich eine Technikkammer T, in welcher alle technischen Komponenten wie Steuerung, Pumpe/n, Filter und dergleichen angeordnet sind.

Die Verbindungsleitung 20 ist aus mehreren Rohrabschnitten 21 und T-förmigen Fittigen 22 (Rohrverbindungsstücken) zusammengesetzt. Die T-förmigen Fittinge 22 weisen beidseitig Anschlüsse zu den Rohrabschnitten 21 und einen hier nach oben weisenden Anschluss zu jeweils einem rohrförmigen Grundkörper 4 auf. An ihrem der Technikkammer abgewandten Ende stützt sich die Verbindungsleitung 20 mittels eines Fußes 23 unten an dem nicht bezeichneten Boden der Box RB ab und in Richtung zur Technikkammer T ist die Verbindungsleitung 20 über einen Verbindungsflansch 24 mit der nicht bezeichneten Trennwand zur Technikkammer T verbunden Im Bereich des Verbindungsflansches 24 ist in der Trennwand ein Durchbruch angeordnet, so dass eine Pumpe und/oder Vakuumpumpe (nicht dargestellt) mit der Verbindungsleitung 20 in Verbindung steht, so dass über die Pumpe/n Luft mit Druck in die Verbindungsleitung einströmt oder Luft abgesaugt wird. In der Technikkammer T befindet sich ein Durchbruch 25, durch welchen Luft ein- bzw. ausströmen kann. Von der Technikkammer T führt weiterhin durch die nicht bezeichnete Trennwand zur Reinigungskammer K ein Luftstromeinlass 25.1.

In der Front der Technikkammer T sind Bedienknöpfe 26 vorgesehen.

Drei hier linke rohrförmige Grundkörper 4 erstrecken sich im Wesentlichen durchgängig von der Verbindungsleitung 20 nach oben. Jeder der drei hier links dargestellten Grundkörper 4 weist im oberen Bereich in Richtung zur Vorderseite und in Richtung zur Rückseite der Box RB nach unten abgewinkelte Vorsprünge V in Form von 90° Winkelstücken 27 auf, in welche UVC-Leuchtmittel 5 nach unten herausragend eingeschraubt sind. Auf diesen Grundkörpern können beispielsweise Jacken oder Kittel gehängt werden. Etwas unterhalb der UVC-Lampen 5 befinden sich in dem rohrförmigen Grundkörper 4 weitere Durchströmöffnungen 4.7.4, die in kreuzförmigen Fittingen 28 ausgebildet sind. Jeder kreuzförmige Fitting 28 weist vier Anschlüsse auf. Ein unterer und ein oberer Anschluss ist mit sich vertikal erstreckenden Rohren des Grundkörpers verbunden, die zweiten seitlichen Anschlüsse sind offen, so dass aus diesen Luft ausströmen kann.

Über die linken Grundkörper 4 mit ihren Vorsprüngen V können beispielsweise Kleidungsstücke wie Jacken oder Kittel gehängt werden.

Rechts sind zwei rohrförmige Grundkörper 4 dargestellt, die aus kürzeren miteinander verbundenen Rohrstücken 21 bestehen. Es erstrecken sich an vertikalen Rohrstücken 21 drei Reihen waagerechter Rohrelemente aus T-förmigen Fittingen 22, 90° Winkelstücken 27 (nach oben abgewinkelt) und kreuzförmigen Fittingen 28 mit jeweils drei nach oben weisenden Vorsprüngen V mit nicht bezeichneten Durchströmöffnungen, in die UVC-Lampen 5 eingeschraubt sind. Diese dienen beispielsweise zur Aufnahme von kleineren Gegenständen wie Atemschutzmasken und dergleichen. Auf die Darstellung der Türen wurde in den Figuren 20 und 21 verzichtet.

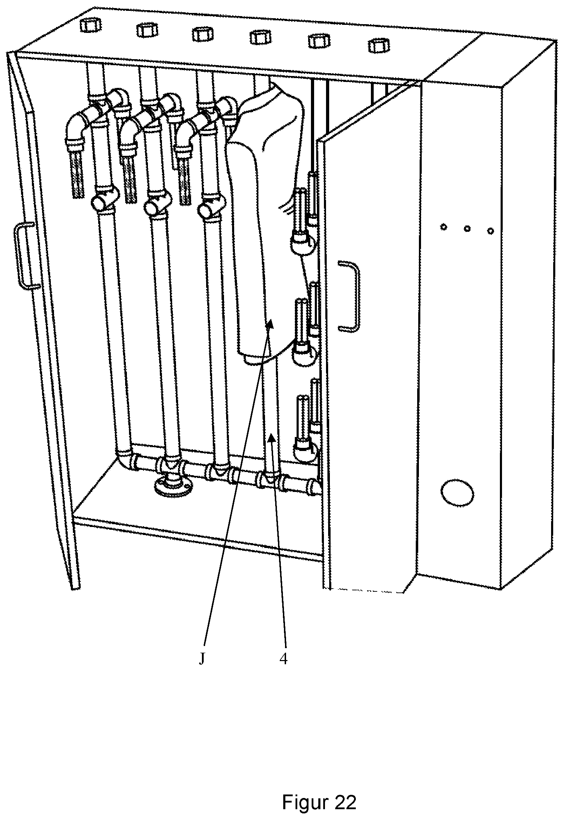

Figur 22 zeigt die Vorrichtung gemäß Figur 20 und 21 aus einer anderen Perspektive mit nicht bezeichneten Türen und einem beispielhaft über einem Grundkörper 4 positionierten Kittel J bzw. einer Jacke.

Es ist selbstverständlich möglich, mehr oder weniger der Grundkörper in einer Box anzuordnen und den Aufbau der Grundkörper sowie die Anzahl und Ausrichtung der Vorsprünge V mit den UVC-Lampen beliebig zu gestalten.

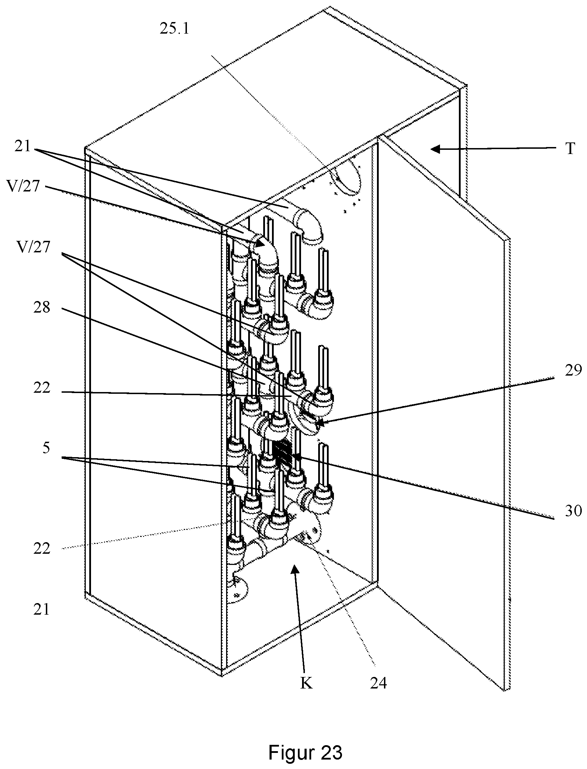

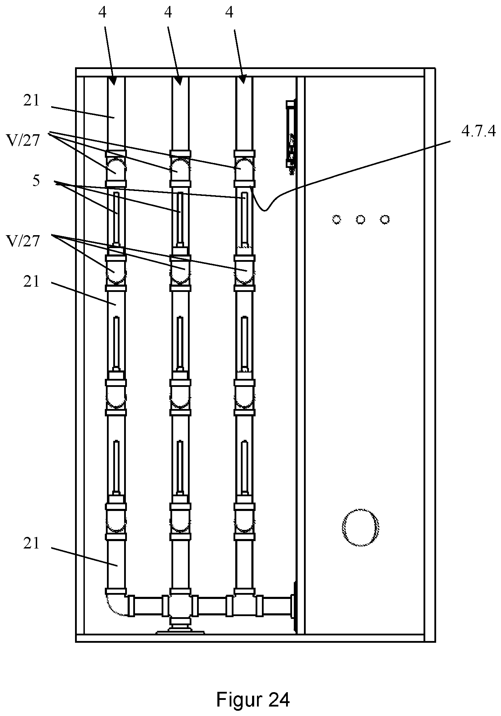

Die Figuren 23 und 24 zeigen eine Vorrichtung die im Wesentlichen wie in den Figuren 20 bis 22 ausgebildet ist, jedoch mit lediglich drei Grundkörpern 4, die übereinander und nebeneinander angeordnete und nach oben weisende Vorsprünge V aufweisen, wie die beiden in den Figuren 20 bis 22 dargestellten rechten Grundkörper 4.

Auch bei der Variante nach den Figuren 23 und 24 sind die Grundkörper 4 aus mehreren Rohrstücken und Rohrverbindungselementen zusammengesetzt. Dies sind Rohrabschnitte 21, T-förmige Fittinge 22, 90° Winkelstücke 27 und kreuzförmige Fittinge 28. Die 90° Winkelstücke 27, welche nach oben abgewinkelt sind, bilden nach oben weisende Vorsprünge V, in welche die UVC-Leuchtmittel 5 eingeschraubt sind.

Über den sich oben befindlichen UVC-Lampen 5 sind hier zusätzliche Winkelstücke 27 angeordnet, deren Durchströmöffnungen 4.7.4 nach unten weisen.

Auch die anderen Winkelstücke 27, welche die UVC-Lampen 5 tragen, weisen nicht bezeichnete Durchströmöffnungen in Richtung zu den UVC-Lampen 5 auf, In der Zwischenwand (nicht bezeichnet) zwischen Reinigungskammer K und Technikkammer T befinden sich ein Luftstromeinlass 25.1, eine Filterbatterie und ein Abluftsystem 29 sowie ein Umlufterzeuger und Heizelement 30 (siehe Figur 23).

Auch hier kann die Anzahl und Gestaltung der Grundkörper 4 sowie die Anzahl, Gestaltung und Ausrichtung der Vorsprünge V und die Anzahl und Ausrichtung der UVC-Lampen 4 nach individuellen Anforderungen gestaltet werden.

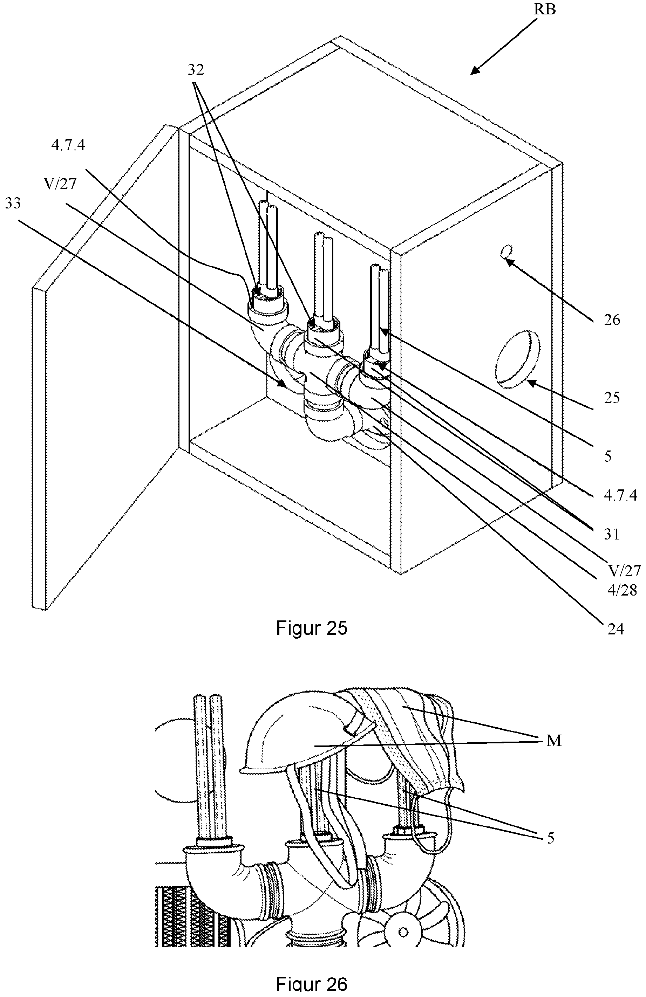

In Figur 25 ist eine Vorrichtung dargestellt, welche lediglich einen kreuzförmigen hohlen Grundkörper 4 aufweist, der durch einen kreuzförmigen Fitting 28 gebildet wird. Es sind drei nach oben weisende Vorsprünge V vorhanden. Der linke und der rechte Vorsprung V werden jeweils durch in seitliche Anschlüsse des Grundkörpers 4 geschraubte und nach oben abgewinkelte Winkelstücke 27 gebildet. Der mittlere Vorsprung V durch den nach oben weisenden Anschluss des Grundkörpers 4.

Die nach oben weisenden Vorsprünge V dieser Rohrverbindungselemente weisen Durchströmöffnungen 4.7.4 auf. In diese Durchströmöffnungen 4.7.4 sind Adapterelemente 31 eingesetzt, welche die UVC-Lampen 5 aufnehmen und die mit Schlitzen 32 versehen sind, durch welche die Luftströmung austreten kann um an den UVC-Lampen vorbeizuströmen. In den drei Adapterelementen 31 der Vorsprünge V sind die UVC-Lampen 5 angeordnet.

In einer Seitenwand der Box RB ist ein Durchbruch 25 angeordnet, der einen Luftstromeinlass bildet. In die Zwischenwand zu einer hier rückseitigen nicht dargestellten Technikkammer mündet ein Umluftkanal 33.

Die Befestigung des Grundkörpers V erfolgt über ein Winkelstück 27, welches an dem nach unten weisenden Anschluss des kreuzförmigen Fittings 27 anschließt und an der Rückwand der Box RB mittels eines Verbindungsflansches 24 befestigt wird. In der Rückwand ist in diesem Bereich ein hier nicht sichtbarer Durchbruch vorgesehen, über welchen in den Grundkörper 4 Luft strömt, die über die Vorsprünge V und die Adapterelemente 31 an den UVC-Lampen 5 vorbeiströmt. An dieser Vorrichtung ist lediglich ein Bedienknopf 26 zum Ein- und Ausschalten vorgesehen. Die Box RB ist mit einer nicht bezeichneten Tür während des Reinigungsvorganges verschließbar.

Figur 26 zeigt eine Teilansicht der Vorrichtung gemäß Figur 25 mit über hier zwei UVC-Lampen 5 aufgenommenen zu reinigenden Gegenständen in Form von Atemschutzmasken M.

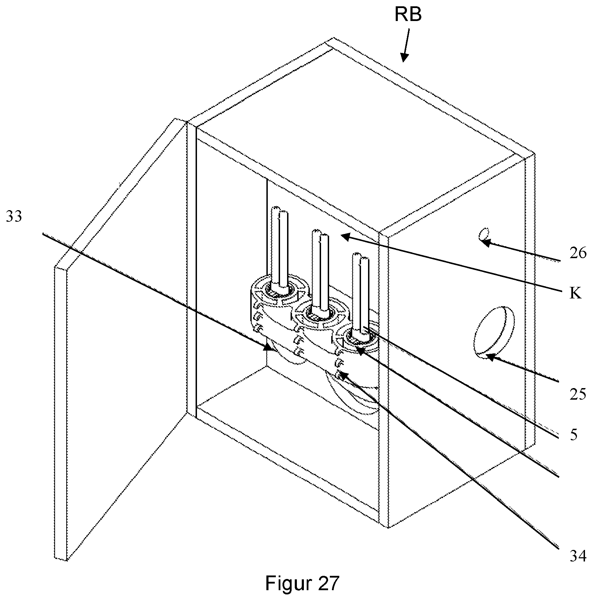

In Figur 27 ist ebenfalls ein in einer Box RB angeordneter Grundkörper 4 dargestellt, der drei nach oben gerichtete kurze Vorsprünge V mit Durchströmöffnungen 4.7.4 und darin sitzenden Adapterelementen 31 mit Schlitzen 32 aufweist. Der Grundkörper 4 wurde hier beispielsweise durch 3D-Druck oder Spritzgießen hergestellt.

Auch hier werden in den Adapterelementen 31 die nach oben weisenden UVC-Lampen 5 befestigt, über welchen beispielsweise Atemschutzmasken positioniert werden können.

An der Vorderseite des Grundkörpers sind mehrere Haken 34 vorgesehen, an welchen zu reinigende Gegenstände wie z.B. Atemschutzmasken aufgenommen werden können.

Die Vorsprünge V wiesen an ihrer Oberseite Durchströmöffnungen 4.7.1 zu den einzelnen Adapterelementen 31 auf.

In einer Seitenwand ist ein Durchbruch 25 vorgesehen, der einen Luftstromeinlass bildet. in die Rückwand der Reinigungskammer K mündet ein Umluftkanal 33. Es ist ebenfalls eine nicht bezeichnete verschließbare Tür vorgesehen. Es ist ebenfalls nur ein Bedienknopf zum starten und beenden des Reinigungsvorganges vorhanden.

Es ist auch möglich, dass der Bedienknopf nur zum Starten des Reinigungsvorganges dient und nach einer bestimmten Zeit dieser Prozess automatisch beendet wird.

Die Adapterelemente, welche in den Figuren 25 und 27 verwendet werden, können auch bei den anderen vorgenannt beschriebenen Lösungen verwendet werden, insbesondere bei den Varianten, die in den Figuren 17 bis 24 dargestellt sind.

Insbesondere durch den modularen Aufbau, der durch die Verwendung von Rohren, Rohrabschnitten, Rohrverbindungselementen möglich ist, kann die Vorrichtungen hinsichtlich der Anzahl und Größe der Grundkörper, der Anzahl und Anordnung der UVC-Lampen variabel gestaltet werden.

Es ist möglich, ein Baukastensystem zur Verfügung zu stellen, mit dem die Vorrichtung individuellen Bedürfnissen anpassbar ist.

Die erfindungsgemäße Lösung ermöglich erstmalig eine vielfältige Möglichkeit der Aufnahme verschiedenster zu reinigenden Gegenständen in einer entsprechenden Vorrichtung sowie deren effiziente Reinigung und Beseitigung von Krankheitserregern.

**Bezugszeichenliste**

| RB | Box |
|---|---|
| 1 | Gehäuse |
| 1a | Korpus |
| 1B | Boden |
| 1D | Deckel |
| 1R | Rückwand |
| 1S | Seitenwand |
| 2 | Kammer |
| 2.2 | zweite Kammer |
| 2.3 | dritte Kammer |
| 2.4 | vierte Kammer |
| 2.2' | Durchbrüche |
| 2.2" | Luftdurchbrüche |
| 3 | Luftzuführung |
| 3' | Einlassregulierung |
| 3.1 | Lufteinlass |
| 3.2 | Lüfter |
| 3.3 | Kammerseitiges Ende der Luftzuführung |
| 3.4 | Heizelement |
| 4 | Grundkörper |
| 4.1 | Haken |
| 4.2 | Trägerelement |
| 4.3 | Trägerelement |
| 4.4 | Anschluss |
| 4.5 | erster Adapter / Grundkörper |
| 4.5.1 | Luftführung |
| 4.5.2 | Austrittsöffnung |
| 4.6 | zweiter Adapter / Grundkörper |
| 4.6' | Aufnahmeelement |
| 4.6.1 | Luftführung |
| 6.6.2 | Austrittsöffnung/Durchbruch |
| 4.6a | Seiten |
| 4.6b | Vorderseite |
| 4.6c | Rückseite |
| 4.7a | Seiten |
| 4.7.2 | Stege |
| 4.7.3 | Durchbrüche |
| 4.7.4 | Durchströmöffungen |
| 4.8.3 | Durchbrüche |
| 4a, 4b | Halbschalen |
| 4c | Platte |
| 4A | sich vertikal erstreckender Bereich |
| 5 | UV-Strahlungsquelle |
| 6 | Absaugeinrichtung |
| 6.1 | Luftauslass |
| 6.1.1 | Austrittsöffnungen |
| 6.2 | Lüfter |
| 7 | Wassertank |
| 7.1 | Heizelement |
| 7.2 | Leitung |
| 7.3 | Verdampfer |
| 8 | Duftkapsel |
| 8.1 | Manueller Regler |
| 9 | Steuerung mit Display |
| 10 | Schuh / Schutzausrüstung |
| 11 | Drucksystem |
| 12 | Querstreben |
| 13 | Aufhängung |
| 14 | Öffnungen |
| 14.2 | Öffnungen |
| 15 | Anschluss |
| 16 | Öffnungen |
| 17 | Schiene |
| 18 | Eintrittsöffnung |
| 19 | Tür |
| 20 | Verbindungsleitung |
| 21 | Rohrabschnitt |
| 22 | T-förmiger Fitting |
| 23 | Fuß |
| 24 | Verbindungsflansch |
| 25 | Durchbruch |
| 25.1 | Luftstromeinlass |
| 26 | Bedienknopf / Bedienknöpfe |
| 27 | 90° Winkelstück |
| 28 | kreuzförmiger Fitting |
| 29 | Filterbatterie und Abluftsystem |
| 30 | Umlufterzeuger und Heizelemente |
| 31 | Adapterelemente |
| 32 | Schlitze |
| 33 | Umluftkanal |
| 34 | Haken |
| A1, A2 | Abdeckungen |
| B | Boden |
| D | Deckel |
| J | Kittel / Jacke |
| L1 | erster Luftstrom |
| L4.6 | zweiter Luftstrom |
| L2 | dritter Luftstrom |
| L4 | vierter Luftstrom |
| M | Atemschutzmasken |
| S1, S2 | Seitenteile |
| V | Vorsprung |
| W1 | Waagerechte Wand zwischen erster und zweiter Kammer |
| W2 | senkrechte Wand zwischen erster und zweiter Kammer |
| W3 | horizontale Wand |
| W4 | horizontale Wand |

## Patentansprüche

1. Vorrichtung zur Reinigung und Desinfizierung wenigstens eines Gegenstandes wie Kleidung und/oder Schutzmasken und/oder persönliche Schutzausrüstung, insbesondere für den medizinischen oder Laborbereich, wobei die Vorrichtung eine von einem Gehäuse umgebene Kammer aufweist, in der ein mindestens ein Grundkörper zur Aufnahme des Gegenstandes angeordnet ist, **dadurch gekennzeichnet, dass** das der Grundkörper einen Hohlraum und an seinem Außenumfang mehrere Durchströmöffnungen zum Hohlraum aufweist, wobei im Bereich mehrerer Durchströmöffnungen jeweils mindestens eine UV-Licht abstrahlende und Ozon erzeugende UV-Strahlungsquelle je Durchströmöffnung angeordnet ist und sich die UV-C-Strahlungsquelle an eine Durchströmöffnung des Grundkörpers anschließt oder zumindest teilweise in die Durchströmöffnung eingreift und dass in den Hohlraum des Grundkörpers mindestens eine Luftzuführung führt, wobei die Luft durch die Durchströmöffnungen an den UV-Strahlungsquellen vorbei in den Gegenstand leitbar ist, wobei
- der Grundkörper mehrere Vorsprünge aufweist, die sich an den Außenumfang des Grundkörpers anschließen, wobei jeweils ein Vorsprung sich an eine Durchströmöffnung anschließt und
∘ wobei eine Vielzahl von sich gegenüberliegenden und übereinander angeordneten Aufnahmen in Form von Vorsprüngen vorgesehen sind
oder
∘ wobei der Grundkörper 4 in Form eines rohrförmigen Hohlkörpers ausgebildet ist, an dessen Umfang sich in einer Ebene jeweils mehrere Vorsprünge V erstrecken und mehrere Vorsprünge V übereinander angeordnet sind, wobei der in der Kammer 2 angeordnete Grundkörper 4 zum Beschicken an seinem unteren Ende drehbar gelagert ist.

2. Vorrichtung nach Anspruch 1, **dadurch gekennzeichnet, dass** mehrere Durchströmöffnungen und Vorsprünge umfangsseitig und/oder übereinander am Grundkörper angeordnet sind, wobei der Grundkörper und die Vorsprünge einen eckigen oder runden Querschnitt aufweisen.

3. Vorrichtung nach Anspruch 1 oder 2, **dadurch gekennzeichnet, dass** der Grundkörper aus der Vorrichtung mittels eines Schienensystems herausziehbar und hineinschiebbar ist.

4. Vorrichtung nacheinem der Ansprüche 1 bis 3, **dadurch gekennzeichnet, dass** die dem Grundkörper zuführbare Luft eine Temperatur zwischen -30 C und 150°C aufweist und in der Vorrichtung ein Druckbereich von 0,1 bar bis 5 bar herrscht.

5. Vorrichtung nach einem der Ansprüche 1 bis 4, **dadurch gekennzeichnet, dass** der Grundkörper austauschbar ist.

6. Vorrichtung nach einem der Ansprüche 1 bis 5, **dadurch gekennzeichnet, dass** mehrere Grundkörper innerhalb einer Kammer angeordnet und untereinander verbunden sind und eine gemeinsame Luftzuführung aufweisen.

7. Vorrichtung nach einem der Ansprüche 1 bis 6, **dadurch gekennzeichnet, dass** der Reinigungszyklus mittels einer Steuereinheit festlegbar oder einstellbar ist und die Parameter wie Temperatur und Druck und/oder Zugabe von Silber-Nanopartikeln festlegbar oder einstellbar sind.

8. Vorrichtung nach einem der Ansprüche 1 bis 7, **dadurch gekennzeichnet, dass** in der Luftzuführung wenigstens ein Duftelement angeordnet ist.

9. Vorrichtung nach einem der Ansprüche 1 bis 8, **dadurch gekennzeichnet, dass** die Vorrichtung einen Wassertank mit einem Heizelement aufweist derart, dass Wasserdampf erzeugbar ist und dass der Wasserdampf über einen Kanal der Luftzuführung zuführbar ist.

10. Verfahren zur Reinigung und Desinfizierung wenigstens eines Gegenstandes, insbesondere wenigstens eines Gegenstandes wie Kleidung und/oder Schutzmasken und/oder persönliche Schutzausrüstung, für den medizinischen, veterinärmedizinischen oder Laborbereich mit einer Vorrichtung nach Anspruch 1, **dadurch gekennzeichnet, dass** der Gegenstand in der Kammer mittels eines Grundkörpers aufgenommen wird und in der Kammer während eines Reinigungszyklus zur Entfernung von Krankheitserregern mittels wenigstens einer, an dem Grundkörper befestigten UV-Lichtquelle UV-C Licht im nicht sichtbaren Wellenlängenbereich und Ozon erzeugt wird und dass die Kammer 2 mit einem Unterdruck und/oder einem Überdruck beaufschlagt wird, wobei mit einem von dem Gehäuse in die Kammer führenden Lufteinlass und einem von der Kammer aus dem Gehäuse führenden Luftauslass ein Luftstrom durch den hohlen Grundkörper erzeugt wird und die Luft über Ausgänge in Form von Durchströmöffnungen aus dem hohl ausgebildeten Grundkörper strömt und an Ozon erzeugenden UV-Strahlungsquellen, die sich an die Durchströmöffnungen anschließen oder zumindest teilweise in die Durchströmöffnungen eingreifen, vorbeiströmt und in den Gegenstand geleitet wird,
wobei der Druck in einem Druckbereich von 0,1 bar bis 5 bar eingestellt wird und dass die Temperatur in der Kammer in einem Temperaturbereich von -30°C bis 150°Ceingestellt wird.

11. Verfahren nach Anspruch 10, **dadurch gekennzeichnet, dass** sich während eines Reinigungszyklus sich Temperatur alternierend innerhalb von 0,5 bis 66 Minuten von 150°C auf -30°C reduziert und anschließend wieder erhöht und/oder dass sich während eines Reinigungszyklus der Druck alternierend innerhalb von 0,1 bis 66 Minuten von 5 bar auf 0,1 bar reduziert und anschließend wieder erhöht.

## Claims

1. A device for cleaning and disinfecting at least one object, such as clothing and/or protective masks and/or personal protective equipment, in particular for medical or laboratory use, wherein the device comprises a chamber enclosed by a housing, in which at least one base body for receiving the object is arranged, **characterised in that** the base body comprises a cavity and, on its outer circumference, a plurality of flow-through openings leading to the cavity, wherein, in the region of several flow-through openings, at least one UV-light-emitting and ozone-generating UV radiation source is arranged for each flow-through opening, and the UV-C radiation source is adjacent to a flow-through opening of the base body or at least partially extends into the flow-through opening, and that at least one air supply leads into the cavity of the base body, whereby the air can be directed through the flow-through openings past the UV radiation sources into the object, wherein
- the base body has a plurality of projections adjoining the outer circumference of the base body, with one projection adjoining each flow-through opening, and
∘ wherein a plurality of mountings, arranged opposite one another and one above the other, are provided in the form of projections
or
∘ wherein the base body 4 is designed in the form of a tubular hollow body, on the circumference of which several projections V extend in a single plane and several projections V are arranged one above the other, the base body 4, which is arranged in the chamber 2, being rotatably mounted at its lower end for supply purposes.

2. The device according to claim 1, **characterised in that** a plurality of flow-through openings and projections are arranged circumferentially and/or one above the other on the base body, wherein the base body and the projections have a polygonal or circular cross-section.

3. The device according to claim 1 or 2, **characterised in that** the base body can be pulled out of and pushed back into the device by means of a rail system.

4. The device according to any one of claims 1 to 3, **characterised in that** the air that can be supplied to the base body has a temperature between -30 °C and 150 °C and a pressure range of 0.1 bar to 5 bar prevails within the device.

5. The device according to any one of claims 1 to 4, **characterised in that** the base body is replaceable.

6. The device according to any one of claims 1 to 5, **characterised in that** a plurality of base bodies are arranged within a chamber and are interconnected, and have a common air supply.

7. The device according to any one of claims 1 to 6, **characterised in that** the cleaning cycle can be defined or adjusted by means of a control unit, and the parameters such as temperature and pressure and/or the addition of silver nanoparticles can be defined or adjusted.

8. The device according to any one of claims 1 to 7, **characterised in that** at least one fragrance element is arranged in the air supply.

9. The device according to any one of claims 1 to 8, **characterised in that** the device comprises a water tank with a heating element such that water vapour can be generated, and **in that** the water vapour can be fed to the air supply via a duct.

10. A method for cleaning and disinfecting at least one object, in particular at least one object such as clothing and/or protective masks and/or personal protective equipment, for use in the medical, veterinary or laboratory sectors, using a device according to claim 1, **characterised in that** the object is held within the chamber by means of a base body and, during a cleaning cycle to remove pathogens, UV-C light in the non-visible wavelength range and ozone are generated within the chamber by means of at least one UV light source attached to the base body, and that the chamber 2 is subjected to a negative pressure and/or a positive pressure, wherein an air flow is generated through the hollow base body by means of an air inlet leading from the housing into the chamber and an air outlet leading from the chamber out of the housing, and the air flows out of the hollow base body via outlets in the form of flow-through openings and passes past ozone-generating UV radiation sources which adjoin the flow-through openings or at least partially extend into the flow-through openings, and is directed into the object,
wherein the pressure is set within a pressure range of 0.1 bar to 5 bar and the temperature in the chamber is set within a temperature range of -30°C to 150°C.

11. The method according to claim 10, **characterised in that**, during a cleaning cycle, the temperature alternately decreases and subsequently increases again between 150°C to -30°C and within 0.5 to 66 minutes, and/or **in that**, during a cleaning cycle, the pressure alternately decreases and subsequently increases again between 5 bar to 0.1 bar within 0.1 to 66 minutes.

## Revendications

1. Dispositif de nettoyage et de désinfection d'au moins un objet, tel que des vêtements et/ou des masques de protection et/ou des équipements de protection individuelle, en particulier pour le domaine médical ou de laboratoire, le dispositif comportant une chambre entourée d'un boîtier dans laquelle au moins un corps de base est placé pour accueillir l'objet, **caractérisé en ce que** le corps de base présente une cavité et à son périmètre extérieur plusieurs ouvertures d'écoulement vers la cavité, étant disposée au niveau de la pluralité d'ouvertures d'écoulement respectivement au moins une source de rayonnement UV émettant des rayons UV et produisant de l'ozone par ouverture d'écoulement et la source de rayonnement UV-C se connectant à une ouverture d'écoulement du corps de base ou empiétant au moins partiellement dans l'ouverture d'écoulement et que, dans la cavité du corps de base, passe au moins une alimentation en air, l'air étant conductible dans l'objet à travers les ouvertures d'écoulement en passant devant les sources de rayonnement UV,
- le corps de base présentant plusieurs saillies qui se connectent à la circonférence extérieure du corps de base, une saillie se connectant respectivement à une ouverture d'écoulement et
∘ une multitude de prises opposées et superposées étant prévues sous forme de saillies ou
∘ le corps de base 4 étant réalisé sous la forme d'un corps creux tubulaire, à la circonférence duquel s'étendent sur un plan respectivement plusieurs saillies V et plusieurs saillies V étant superposées, le corps de base 4 disposé dans la chambre 2 s'appuyant en rotation à son extrémité inférieure pour l'alimentation.

2. Dispositif selon la revendication 1, **caractérisé en ce que** plusieurs ouvertures d'écoulement et saillies sont disposées en circonférence et/ou superposées sur le corps de base, le corps de base et les saillies présentant une section transversale angulaire ou ronde.

3. Dispositif selon la revendication 1 ou 2, **caractérisé en ce que** le corps de base peut être extrait du dispositif et glissé dans celui-ci au moyen d'un système de rails.

4. Dispositif selon l'une des revendications 1 à 3, **caractérisé en ce que** l'air pouvant être apporté dans le corps de base a une température comprise entre - 30 °C et 150 °C et qu'une plage de pression de 0,1 bar à 5 bar règne dans le dispositif.

5. Dispositif selon l'une des revendications 1 à 4, **caractérisé en ce que** le corps de base est interchangeable.

6. Dispositif selon l'une des revendications 1 à 5, **caractérisé en ce que** plusieurs corps de base sont disposés à l'intérieur d'une chambre et reliés entre eux et présentent une alimentation en air commune.

7. Dispositif selon l'une des revendications 1 à 6, **caractérisé en ce que** le cycle de nettoyage peut être défini ou réglable au moyen d'une unité de commande et que les paramètres tels que la température et la pression et/ou l'ajout de nanoparticules d'argent peuvent être définis ou réglés.

8. Dispositif selon l'une des revendications 1 à 7, **caractérisé en ce qu'**au moins un élément odorant est placé dans l'alimentation en air.

9. Dispositif selon l'une des revendications 1 à 8, **caractérisé en ce que** le dispositif comporte un réservoir d'eau avec un élément chauffant de telle sorte que de la vapeur d'eau puisse être générée et que la vapeur d'eau puisse être acheminée vers l'alimentation en air par un canal.

10. Procédé de nettoyage et de désinfection d'au moins un objet, en particulier d'au moins un objet tel que des vêtements et/ou des masques de protection et/ou des équipements de protection individuelle, pour le domaine médical, vétérinaire ou de laboratoire, avec un dispositif selon la revendication 1, **caractérisé en ce que** l'objet est pris dans la chambre au moyen d'un corps de base et que, dans la chambre, pendant un cycle de nettoyage, afin d'éliminer les agents pathogènes, au moyen d'au moins une source de lumière UV fixée au corps de base, de la lumière UV-C dans la plage de longueur d'onde non visible et de l'ozone sont générés et que la chambre 2 est soumise à une pression négative et/ou à une surpression, avec une entrée d'air allant du boîtier dans la chambre et une sortie d'air de la chambre provenant du boîtier, un flux d'air est généré à travers le corps de base creux et l'air circule à travers des sorties se présentant sous forme d'ouvertures d'écoulement du corps de base de conformation creuse et passe devant des sources de rayonnement UV générant de l'ozone, qui se connectent aux ouvertures d'écoulement ou empiètent au moins partiellement dans les ouvertures d'écoulement, et est conduit dans l'objet,
la pression étant réglée dans une plage de pression de 0,1 bar à 5 bar, et que la température dans la chambre est réglée dans une plage de température de -30 °C à 150 °C.

11. Procédé selon la revendication 10, **caractérisé en ce que**, pendant un cycle de nettoyage, la température diminue alternativement de 150 °C à -30 °C en 0,5 à 66 minutes, puis augmente à nouveau et/ou que, pendant un cycle de nettoyage, la pression diminue alternativement de 5 bar à 0,1 en 0,1 à 66 minutes puis augmente à nouveau.
